# EUROPEAN PATENT APPLICATION

(11) **EP 4 736 859 A1**
(43) Date of publication of application: **06.05.2026**
(21) Application number: 24210388.5
(22) Date of filing: 01.11.2024
(51) Int. Cl.: A61K 31/708, A61K 31/505, A61K 31/513, A61K 31/635, A61K 31/7072, A61K 45/06, A61P 31/04

(54) **ANTIBIOTIC COMPOSITIONS**

(71) Applicant: University of Galway, H91 TK33 Galway (IE)
(72) Inventor: O'Gara, James P, Galway, H91 TK33 (IE); Nolan, Aaron C, Galway, H91 TK33 (IE); Zeden, Merve S, Galway, H91 T33 (IE); Campbell, Christopher, Galway, H91 TK33 (IE)
(74) Representative: Barker Brettell LLP

(57) **Abstract**

The invention relates to an antibiotic composition comprising or consisting of: a purine nucleoside selected from guanosine and xanthosine, or a combination thereof; and an inhibitor of thymidine and/or pyrimidine biosynthesis. A further combination with a Beta-lactam antibiotic can be provided. The invention further relates to associated kits, methods and uses in treatment.

## Description

### Technical Field

The present invention relates to antibiotic compositions suitable for the treatment of bacterial infections, particularly wherein the infections are caused by antibiotic-resistant bacteria, and related products, uses, and methods of treatment.

### Introduction

Antimicrobial resistance (AMR) is a troubling burden on worldwide health, and it is predicted that by 2050, 10 million deaths per year could be caused by pathogens with increasing rates of AMR. *Staphylococcus aureus* infections are one of the leading pathogens for death toll each year categorised in the ESKAPE pathogen category, and of these deaths majority are caused by methicillin resistant *S. aureus* (MRSA). MRSA is resistant to majority of β-lactam antibiotics on the market which is considered the gold standard for treating *S. aureus* infections, although there are alternative treatment strategies for MRSA such as vancomycin and daptomycin, they have high treatment failure rates.

The rising levels of increasing resistance seen due to MRSA infections is leading to novel treatment strategies being considered to treat potentially life-threatening bacterial infections with dwindling choices of antibiotics for therapeutic intervention. The imminent threat of AMR infections has called upon novel strategies such as bacteriophage therapy, new antibiotic development, identifying novel therapeutic targets and antibiotic adjuvants. Adjuvants can re-sensitise MRSA to antibiotic treatment as shown recently by our group by disrupting cyclic-di-adenosine-monophosphate (c-di-AMP) levels in MRSA increasing beta-lactam susceptibility (1, 2).

Nolan et al. (2023. mBio 14:e02478-22. https://doi.org/10.1128/mbio.02478-22 - herein incorporated by reference) is a research article that investigates how purine nucleosides interfere with c-di-AMP levels and act as adjuvants to re-sensitize MRSA to β-lactam antibiotics. It demonstrates how using purine nucleosides as adjuvants to increase the susceptibility of clinically important pathogens to β-lactams has the potential to facilitate new treatments with lower antibiotic doses and with drug combinations that are toxic at higher concentrations. *In vitro* kill curves revealed significant bactericidal activity using combinations of guanosine and oxacillin (OX) at concentrations up to 128 µg/mL, which are within the range of flucloxacillin intravenously administered to human patients (100-200 mg/kg/day). While the emergence of OX/Gua suppressor mutants appeared to be significantly reduced at higher oxacillin concentrations (as evidenced by the absence of re-growth in kill curve cultures), this remains a potential barrier to the use of nucleosides as β-lactam adjuvants.

Therefore, further new treatments are required to optimise purine nucleosides as adjuvants and to further increase the effectiveness of anti-microbials, such as β-lactams.

### Summary of the Invention

According to a first aspect of the invention, there is provided an antibiotic composition comprising or consisting of:
a β-lactam antibiotic;
a purine nucleoside selected from guanosine and xanthosine, or a combination thereof; and
an inhibitor of thymidine and/or pyrimidine biosynthesis.

According to another aspect of the invention, there is provided an antibiotic composition comprising or consisting of:
guanosine or xanthosine; and
an inhibitor of thymidine and/or pyrimidine biosynthesis.

The antibiotic composition may be for killing or inhibiting the growth of a targeted bacterium.

The inventors have identified that it is possible to restore susceptibility to a β-lactam antibiotic in a β-lactam-resistant bacterium by interfering with its endogenous thymidine biosynthetic pathway.

Advantageously, the present invention may reduce resistance to β-lactam antibiotics in planktonic bacteria and in biofilms. Further advantageously, the present invention can significantly reduce the minimum inhibitory concentration (MIC) of a β-lactam antibiotic by exploiting newly identified synergies between
1. the β-lactam antibiotic, a purine nucleoside, and an inhibitor of thymidine biosynthesis.
2. the purine nucleoside guanosine and sulfamethoxazole, which is required for production of tetrahydrofolate, a co-factor in thymidine biosynthesis.
3. a triple combination of the purine nucleoside guanosine, sulfamethoxazole and 5-fluorouracil, which interferes with pyrimidine biosynthesis.

Further advantageously, the present invention can significantly enhance killing of viable planktonic bacteria by combinations of antibiotics (e.g., β-lactams, SMX, TMP, 5-FU and 5-FUrd) over 12-24 hours at concentrations of the agents that would normally be insufficient when used alone.

A further advantage has been identified in that guanosine and/or xanthosine combined with a thymidine synthesis inhibitor, such as 5-FU, and/or a pyrimidine synthesis inhibitor, such as SMX, demonstrates a surprisingly high level of anti-bacterial activity, particularly anti-MRSA killing activity.

### The Purine Nucleoside

The purine nucleoside may comprise guanosine. In another embodiment, the purine nucleoside may comprise xanthosine. The purine nucleoside may comprise or consist of a combination of guanosine and xanthosine. In all embodiments, the purine nucleoside may not comprise adenosine.

In one embodiment, the purine nucleoside is not a derivative of guanosine or xanthosine. Derivatives of guanine, xanthine, uridine and adenosine combined with oxacillin in kill curve assays with MRSA strain JE2 were also investigated and did not exhibit the same antibacterial effects. These derivates were guanosine monophosphate, acyclovir, caffeine (xanthine derivative) and trifluridine, which did not act synergistically with oxacillin as well as 3-methylxanthine and 6-thioguanine which antagonised the activity of oxacillin.

The purine nucleoside may be provided in the composition at a therapeutically effective concentration. The purine nucleoside may be provided in the composition at a concentration up to about 960mg/kg or consumed in solution up to 1g/L. The concentration may be a therapeutically effective amount up to 960 mg/kg or up to 1g/L in a solution. The purine nucleoside may be provided at a concentration from about 100 mg/kg to 960 mg/kg in solid form or from about 0.1g/L to 1g/L in liquid form. In another embodiment, the purine nucleoside may be provided at a concentration from about 500 mg/kg to 960 mg/kg in solid form or from about 0.5g/L to 1g/L in liquid form. In regard to embodiments wherein the purine nucleoside comprises or consist of a combination of more than one type of purine nucleoside, these concentration values refer to the total amount of purine nucleoside in the composition.

The purine nucleoside may be provided in an amount of at least 33 % of the composition. The guanosine analogue may be provided in an amount of from about 30% to about 90 % w/v of the composition. The guanosine analogue may be provided in an amount of from about 30% to about 87 % w/v of the composition. In regard to embodiments wherein the purine nucleoside comprises or consist of a combination of more than one type of purine nucleoside, these values refer to the total amount of purine nucleoside in the composition.

### The β-lactam antibiotic

The β-lactam antibiotic may be a molecule comprising a β-lactam ring and wherein the molecule is capable of preferentially binding to DD-transpeptidases (also known as penicillin binding proteins (PBPs)). The β-lactam antibiotic of the composition may comprise methicillin, oxacillin, nafcillin, cefoxitin, cefotaxime, cefaclor, penicillin G, cloxacillin, or a variant thereof. The β-lactam antibiotic may comprise methicillin, or a variant thereof, for example a functional derivative thereof. The β-lactam antibiotic may comprise a penicillin, or a variant thereof, for example a functional derivative thereof. The β-lactam antibiotic may comprise oxacillin, or a variant thereof, for example a functional derivative thereof. The β-lactam antibiotic may comprise a penicillin, a cephalosporin, a carbapenem, or a variant thereof. The β-lactam antibiotic may comprise or consist of a combination of more than one type of β-lactam antibiotic described herein.

The β-lactam antibiotic may be capable of inhibiting or killing bacteria, such as *S*. *aureus,* when used alone, and the bacteria does not synthesise a β-lactamase and/or does not synthesise penicillin-binding proteins modified to resist binding of the antibiotic.

The β-lactam antibiotic may be provided in the composition at therapeutically effective concentration. The skilled person will be aware of effective dosing levels of β-lactam antibiotics in adults and children, for example by reference to the literature, such as Abdul et al. (Frontiers in Pharmacology, V.13, 2022. DOI=10.3389/fphar.2022.964005, ISSN=1663-9812), which is herein incorporated by reference. The therapeutically effective concentration of the β-lactam antibiotic may be substantially reduced when used in combination with the purine nucleoside, and the inhibitor of thymidine or pyrimidine biosynthesis. Therefore, the β-lactam antibiotic may be provided in the composition at therapeutically effective concentration, which is therapeutically effective as part of the combination of the β-lactam antibiotic with the purine nucleoside, and the inhibitor of thymidine or pyrimidine biosynthesis. The β-lactam antibiotic, such as oxacillin, may be provided in the composition at a concentration of from about 25 mg/kg to about 300 mg/kg. In another embodiment, the β-lactam antibiotic, such as oxacillin, is provided at a concentration of about 50-200 mg/kg. In another embodiment, the β-lactam antibiotic, such as oxacillin, is provided at a concentration of about 75mg/kg. In regard to embodiments wherein the β-lactam antibiotic comprises or consists of a combination of more than one type of β-lactam antibiotic, these concentration values refer to the total amount of β-lactam antibiotics in the composition.

The β-lactam antibiotic may be provided in an amount of at least 7 % of the composition. The β-lactam antibiotic may be provided in an amount of from about 7 to about 33 % w/v of the composition. In regard to embodiments wherein the β-lactam antibiotic comprises or consists of a combination of more than one type of β-lactam antibiotic, these values refer to the total amount of β-lactam antibiotics in the composition.

In one embodiment, the β-lactam antibiotic of the composition comprises at least one type of β-lactam antibiotic to which the target bacterium is resistant, when the antibiotic is used alone.

### The inhibitor of thymidine and/or pyrimidine biosynthesis

The skilled person will recognise that there is some overlap/interaction between the thymidine and pyrimidine biosynthesis pathways. Therefore, an inhibitor of one pathway may also inhibit the other pathway directly or indirectly. An inhibitor of the pyrimidine biosynthesis pathway may be a more direct or specific inhibitor of the pyrimidine biosynthetic pathway relative to the thymidine biosynthesis pathway, and vice versa. In one embodiment, both pathways are inhibited by the same inhibitor.

The inhibitor of thymidine and/or pyrimidine biosynthesis may comprise an agent that inhibits thymidine and/or pyrimidine biosynthesis in bacteria, such as a target bacterium of the antibiotic composition. The inhibition may be direct inhibition on the synthesis pathway, or indirect inhibition (i.e. not direct inhibition on the pathway).

The inhibitor of thymidine and/or pyrimidine biosynthesis may comprise an agent that inhibits thymidylate synthase. Such an inhibition may be direct inhibition, or indirect inhibition, such as interfering with the production of a co-factor for thymidylate synthase (e.g. tetrahydrofolate). The inhibitor of thymidine and/or pyrimidine biosynthesis may comprise an agent that inhibits an enzyme of the thymidine and/or pyrimidine biosynthesis pathway, either directly or indirectly. For example, by inhibition of one or more of dihydroorotate dehydrogenase (DHODH), aspartate transcarbamoylase (ATCase), dihydrofolate reductase (DHFR), carbamoyl phosphate synthetase II (CPS II), thymidylate synthase (TS), orotindine 5'-monophosphate decarboxylase (ODCase), and folate metabolism.

By way of example, impairment or inhibition of thymidine biosynthesis may be achieved by the inhibitor of thymidine and/or pyrimidine biosynthesis by enzyme substrate competition, or by scavenging necessary enzyme cofactors, or by a combination of the two, and/or by other mechanisms. The inhibitor of thymidine and/or pyrimidine biosynthesis may comprise a compound that impairs *de novo* thymidine biosynthesis by competing with its starting substrate uracil, for example 5-fluorouracil (5-FU, also known in the art as fluorouracil or Adrucil), or 5-fluorouridine (5-FUrd). Alternative inhibitors of thymidine and/or pyrimidine biosynthesis may comprise other uridine analogues, such as floxuridine/5-fluorodeoxyuridine or 5'deoxy-5-fluorouridine.

Alternatively or additionally, the inhibitor of thymidine and/or pyrimidine biosynthesis may comprise a sulphonamide antibiotic, such as sulfamethoxazole (SMX). The sulphonamide antibiotic, such as SMX may compete with the substrate of the enzyme dihydropteroate synthase, thereby preventing thymidine processing and thus impairing biochemical pathways that rely on thymidine-based metabolites. Alternatively or additionally, the inhibitor of thymidine and/or pyrimidine biosynthesis may comprise a compound, such as trimethoprim (TMP), that competes with the substrate of the enzyme dihydrofolate reductase, thereby similarly preventing thymidine processing and thus similarly impairing biochemical pathways that rely on thymidine-based metabolites.

The inhibitor of thymidine and/or pyrimidine biosynthesis may be selected from 5-fluorouracil (5-FU), 5-fluorouridine (5-FUrd), sulfamethoxazole (SMX), trimethoprim (TMP), and combinations thereof, or variants thereof. The inhibitor of thymidine and/or pyrimidine biosynthesis may comprise a sulphonamide antibiotic. The inhibitor of thymidine and/or pyrimidine biosynthesis may comprise 5-fluorouracil, or a variant thereof, for example a functional derivative thereof. The inhibitor of thymidine and/or pyrimidine biosynthesis may comprise 5-fluorouridine, or a variant thereof, for example a functional derivative thereof. The inhibitor of thymidine and/or pyrimidine biosynthesis may comprise sulfamethoxazole, or a variant thereof, for example a functional derivative thereof. The inhibitor of thymidine and/or pyrimidine biosynthesis may comprise trimethoprim, or a variant thereof, for example a functional derivative thereof.

The inhibitor of thymidine and/or pyrimidine biosynthesis may comprise or consist of a combination of more than one type of inhibitor of thymidine and/or pyrimidine biosynthesis according to this specification. For example, the inhibitor of thymidine and/or pyrimidine biosynthesis may comprise a combination of 5-FU and SMX (or other sulphonamide antibiotics), or of variants (for example, functional derivatives) or either compound, or of both compounds. As a further example, the inhibitor of thymidine and/or pyrimidine biosynthesis may comprise a combination of sulfamethoxazole and trimethoprim (SMX-TMP, also known in the art as sulfamethalazole or sulfisomezole), or of variants (for example, functional derivatives) of either compound, or of both compounds. The molar ratio of sulfamethoxazole:trimethoprim in the composition may be from about 75 to about 100 mg/kg for sulfamethoxazole and 15 to about 20 mg/kg for trimethoprim. The skilled person will be capable of identifying safe and therapeutically effective levels of sulfamethoxazole and/or trimethoprim.

The inhibitor of thymidine and/or pyrimidine biosynthesis may be provided in the composition at a concentration of from about 7% to about 33% w/v. Preferably, the inhibitor of thymidine and/or pyrimidine biosynthesis is provided at a concentration of about 33%. Preferably, in embodiments wherein the inhibitor of thymidine and/or pyrimidine biosynthesis comprises 5-FU or 5-FUrd, the anti-thymidine agent may be provided in the composition at a concentration of from about 15 mg/kg to about 240 mg/kg. In regard to embodiments wherein the inhibitor of thymidine and/or pyrimidine biosynthesis comprises or consists of a combination of more than one type of inhibitor of thymidine and/or pyrimidine biosynthesis, these concentration values refer to the total amount of inhibitors of thymidine and/or pyrimidine biosynthesis in the composition.

The inhibitor of thymidine and/or pyrimidine biosynthesis may be provided in an amount of at least 7 % of the composition. The inhibitor of thymidine and/or pyrimidine biosynthesis may be provided in an amount of from about 7 to about 33 % w/v of the composition. In regard to embodiments wherein the inhibitor of thymidine and/or pyrimidine biosynthesis comprises or consists of a combination of more than one type of inhibitor of thymidine and/or pyrimidine biosynthesis, these values refer to the total amount of inhibitors of thymidine and/or pyrimidine biosynthesis in the composition.

### The antibiotic composition

The antibiotic composition may be for administration alone, or in combination with at least one other medicament.

The antibiotic composition may comprise a carrier as an additional component. The carrier may be a pharmaceutically suitable carrier. The composition may be formulated as a topical, systemic, or localised treatment, or a combination thereof. The composition may be formulated for enteral (e.g. oral, rectal), parenteral (e.g. intraosseous, intravitreal, intra-articular, intradermal, subcutaneous, intrathecal, epidural, intravenous, intracardiac, intramuscular, or intraperitoneal), buccal, sublingual, transnasal, opthalmic, urogenital/vaginal, or transdermal administration, or for administration by inhalation or by implantation (e.g. of a transdermal implant), or a combination thereof. The composition may be formulated as cream, ointment, gel, paste, patch, liquid, lotion, powder, tablet, pill, capsule, pastille, lozenge, balm, beverage, emulsion, paste, aerosol, spray, eye or ear drops, pessary, suppository, hydrogel, enema, infusion, impregnated or coated implant. The composition may be administered indirectly, e.g. *via* catheter or cannula.

The person skilled in the art will be acquainted with various formulations and their degree of suitability for a given route of administration and thus match the formulation to the desired administration route e.g. sublingual administration may be best suited to a powder-based formulation. The person skilled in the art will also be able to adapt common formulation preparation techniques to achieve a composition substantially according to the specification that is suited to the desired route of administration.

The antibiotic composition may be sterile, for example the antibiotic composition may be sterilised during manufacture or before administration.

The composition may be provided in a therapeutically effective amount. The composition may be provided in a dose that is sufficient to kill or inhibit the growth or a targeted bacterial cell, for example *in vivo* in a subject. The therapeutically effective amount of the composition may comprise at least one component in an amount that is lower than the therapeutically affective amount of that component when said component is used without the other components of the composition. When provided in combination with the other components of the composition of the invention, the MIC of at least one component of the composition may be lower than the literature MIC of that component used alone, i.e. lower than the MIC of that component when administered not as part of the composition of the invention.

The antibiotic formulation may be provided in the form of a kit of parts.

The antibiotic formulation may be provided as a formulated dose (i.e. a formulation that is ready to be administered), or alternatively as a pre-formulation (e.g. a formulation that requires dissolution and/or dilution prior to administration.)

In one embodiment, the β-lactam antibiotic, the purine nucleoside, and the inhibitor of thymidine or pyrimidine biosynthesis, are provided in a ratio of about 1:1:1 w/w, or up to 30% variation thereof for one or more of the components. In another embodiment, the β-lactam antibiotic, the purine nucleoside, and the inhibitor of thymidine or pyrimidine biosynthesis, are provided in a ratio of about 1:1:1 w/w, or up to 20% variation thereof for one or more of the components. In another embodiment, the β-lactam antibiotic, the purine nucleoside, and the inhibitor of thymidine or pyrimidine biosynthesis, are provided in a ratio of about 1:1:1 w/w, or up to 10% variation thereof for one or more of the components. In another embodiment, the β-lactam antibiotic, the purine nucleoside, and the inhibitor of thymidine or pyrimidine biosynthesis, are provided in a ratio of about 1:1:1 w/w.

In another embodiment, the β-lactam antibiotic, the purine nucleoside, and the inhibitor of thymidine or pyrimidine biosynthesis, are provided in a ratio of about 0.8-1.2 : 0.8-1.2 : 0.8-1.2 w/w.

In another embodiment, the β-lactam antibiotic, the purine nucleoside, and the inhibitor of thymidine or pyrimidine biosynthesis, are provided in a ratio from about 1:1:1 to about 1:14:1. In another embodiment, the β-lactam antibiotic, the purine nucleoside, and the inhibitor of thymidine or pyrimidine biosynthesis, are provided in a ratio of about 1:13:1 or 1:14:1. The molar ratio of β-lactam antibiotic : purine nucleoside : anti-thymidine or pyrimidine biosynthesis agent in the composition may be approximately 6.5:87:6.5.Where the concentration of a component is provided, this may refer to the concentration of the component in the composition including the carrier, or to the concentration of the component in the composition excluding the carrier. The concentration of a component may refer to the concentration of the component in the composition including the carrier. The provided concentration of a component may refer to its concentration in a formulated dose, or to its concentration in a pre-formulation.

In some embodiments, all the components of the composition are provided already mixed. In such embodiments, the composition may be provided as a formulated dose, or as a pre-formulation. In alternative embodiments, at least one component of the composition is provided physically separated from at least one other component, so that the components may be freshly mixed prior to administration. The person skilled in the art will recognise that, in these alternative embodiments, kits may be provided wherein all the components of the composition are included (for ease of usage), and further wherein at least one component is physically separated from at least one other (e.g. for stability).

In some embodiments wherein at least one component of the composition is provided physically separated from at least one other component, all the components of the combination are pre-mixed before administration, that is to say, administration of all components is simultaneous. In other embodiments, the components of the composition are administered separately, e.g. sequentially or concurrently.

In embodiments wherein the β-lactam antibiotic comprises oxacillin, methicillin, nafcillin, cefotaxime, cefaclor, penicillin G, or cloxacillin, or a combination thereof, the preferred purine nucleoside comprises guanosine. In an embodiment wherein the β-lactam antibiotic comprises cefoxitin, the preferred purine nucleoside comprises xanthosine.

In one embodiment, the composition comprises at least the MIC of each component in its combination. Advantageously, this will be significantly lower than when the components are used as monotherapy. In one embodiment, the composition may comprise a combination of components as described in Table 4, wherein the concentrations are at least the MIC of each component in its combination and wherein the guanosine is substituted with xanthosine. In these embodiments, the guanosine of Table 4 may be adjusted in concentration to an amount of 100-600 µg/ml guanosine or may be substituted with 100-600 µg/ml xanthosine.

The skilled person will recognise that the MIC, or more, of the components in their combinations may be provided, and optionally the maximum concentration of each component may be limited to a level that is less than the concentration that is considered to be clinically toxic to a mammal, such as a human; further optionally, the maximum concentration of each component may be limited to the concentration at which that component is typically used as a monotherapy (i.e. not in combination with any other component of the composition). Therefore, in embodiments, the maximum concentration of 5-FU in combination with at least one other component in the composition according to the invention may be about 15 mg/kg/day, wherein the total dose administered to the subject daily does not exceed about 1 g 5-FU; the maximum concentration of oxacillin in combination with at least one other component in the composition according to the invention may be about 200 mg/kg/day, or about 100 mg/kg/day; the maximum concentration of TMP in combination with at least one other component in the composition according to the invention may be about 4 mg/kg/day, wherein the total dose administered to the subject daily does not exceed about 200 mg TMP; the maximum concentration of SMX in combination with at least one other component in the composition according to the invention may be about 18 mg/kg/day, wherein the total dose administered to the subject daily does not exceed about 800 mg SMX; the maximum concentrations of TMP and SMX, when administered as TMP-SMX, in combination with at least one other component in the composition according to the invention, may be about 4 mg/kg/day TMP and about 18 mg/kg/day SMX (up to a limit of about 200 mg/day and about 800 mg/day, respectively); wherein in all such cases: 'kg' refers to the body weight of the subject to be administered, 'day' or 'daily' refer to any 24-hour period, and the 'maximum concentration administered' and 'total dose administered daily' for each component in the composition are less than the concentration or daily dose, respectively, that are considered to be clinically toxic to a mammal, such as a human.

In one embodiment, the composition comprises about 100-1000 µg/ml guanosine, about 0.1-1 µg/ml of oxacillin and about 0.1-1 µg/ml 5-FU. In another embodiment, the composition comprises about 100-600 µg/ml guanosine, about 0.1-1 µg/ml of oxacillin and about 0.1-1 µg/ml 5-FU. In another embodiment, the composition comprises about 200 µg/ml guanosine, about 0.5 µg/ml of oxacillin and about 0.5 µg/ml 5-FU.

In one embodiment the antibiotic composition comprises about 10-20 µg/ml oxacillin, and about 50-100 µg/ml SMX. In another embodiment the antibiotic composition comprises about 16 µg/ml oxacillin, and about 64 µg/ml SMX. In another embodiment the antibiotic composition comprises about 50-100 µg/ml oxacillin, and about 10-20 µg/ml SMX. In another embodiment the antibiotic composition comprises about 64 µg/ml oxacillin, and about 16 µg/ml SMX. In one embodiment the antibiotic composition comprises about 0.05-0.2 µg/ml oxacillin, and about 0.1-0.5 µg/ml TMP. In another embodiment the antibiotic composition comprises about 0.12 µg/ml oxacillin, and about 0.25 µg/ml TMP. In one embodiment the antibiotic composition comprises about 0.1-0.5 µg/ml oxacillin, and about 0.005-0.02 µg/ml 5-FUrd. In one embodiment the antibiotic composition comprises about 0.25 µg/ml oxacillin, and about 0.012 µg/ml 5-FUrd.

In one embodiment the antibiotic composition comprises about 100-1000 µg/ml guanosine, about 0.03-0.12 µg/ml oxacillin, and about 10-20 µg/ml SMX. In one embodiment the antibiotic composition comprises about 100-600 µg/ml guanosine, about 0.03-0.12 µg/ml oxacillin, and about 10-20 µg/ml SMX. In another embodiment the antibiotic composition comprises about 200 µg/ml guanosine, about 0.06 µg/ml oxacillin, and about 16 µg/ml SMX. In one embodiment the antibiotic composition comprises about 100-1000 µg/ml guanosine, about 0.1-2 µg/ml oxacillin, and about 4-16 µg/ml SMX. In another embodiment the antibiotic composition comprises about 100-600 µg/ml guanosine, about 0.1-2 µg/ml oxacillin, and about 4-16 µg/ml SMX. In another embodiment the antibiotic composition comprises about 200 µg/ml guanosine, about 0.5 µg/ml oxacillin, and about 8 µg/ml SMX. In one embodiment the antibiotic composition comprises about 100-1000 µg/ml guanosine, about 0.5-2 µg/ml oxacillin, and about 2-8 µg/ml SMX. In one embodiment the antibiotic composition comprises about 100-600 µg/ml guanosine, about 0.5-2 µg/ml oxacillin, and about 2-8 µg/ml SMX. In another embodiment the antibiotic composition comprises about 200 µg/ml guanosine, about 1 µg/ml oxacillin, and about 4 µg/ml SMX.

In one embodiment the antibiotic composition comprises about 100-1000 µg/ml guanosine, about 0.01-0.1 µg/ml oxacillin, and about 0.1-0.5 µg/ml TMP. In another embodiment the antibiotic composition comprises about 100-600 µg/ml guanosine, about 0.01-0.1 µg/ml oxacillin, and about 0.1-0.5 µg/ml TMP. In another embodiment the antibiotic composition comprises about 200 µg/ml guanosine, about 0.03 µg/ml oxacillin, and about 0.25 µg/ml TMP. In one embodiment the antibiotic composition comprises about 100-1000 µg/ml guanosine, about 0.05-0.3 µg/ml oxacillin, and about 0.05-0.3 µg/ml TMP. In another embodiment the antibiotic composition comprises about 100-600 µg/ml guanosine, about 0.05-0.3 µg/ml oxacillin, and about 0.05-0.3 µg/ml TMP. In another embodiment the antibiotic composition comprises about 200 µg/ml guanosine, about 0.12 µg/ml oxacillin, and about 0.12 µg/ml TMP. In one embodiment the antibiotic composition comprises about 100-400 µg/ml guanosine, about 0.5-2 µg/ml oxacillin, and about 0.03-0.12 µg/ml TMP. In another embodiment the antibiotic composition comprises about 100-600 µg/ml guanosine, about 0.5-2 µg/ml oxacillin, and about 0.03-0.12 µg/ml TMP. In another embodiment the antibiotic composition comprises about 200 µg/ml guanosine, about 1 µg/ml oxacillin, and about 0.06 µg/ml TMP.

In one embodiment the antibiotic composition comprises about 100-1000 µg/ml guanosine, about 0.03-0.12 µg/ml oxacillin, and about 0.001-0.01 µg/ml 5-FUrd. In another embodiment the antibiotic composition comprises about 100-600 µg/ml guanosine, about 0.03-0.12 µg/ml oxacillin, and about 0.001-0.01 µg/ml 5-FUrd. In another embodiment the antibiotic composition comprises about 200 µg/ml guanosine, about 0.06 µg/ml oxacillin, and about 0.003 µg/ml 5-FUrd.

In embodiments wherein the antibiotic composition is used to treat or inhibit a biofilm, the concentration of at least one of the components of the antibiotic composition may vary from (such us by being greater than) the concentrations hereinabove. For example, in such embodiments, the concentration of the β-lactam antibiotic in the composition may be about 1.2 times, about 1.5 times, about 2 times, about 2.5 times, or about 3 times the otherwise preferred concentration of the β-lactam antibiotic in the composition, and the concentrations of the other components of the composition is not varied to their respective preferred concentration. As an additional example, in the same such embodiments, the concentration of each of the purine nucleosides, the β-lactam antibiotic, and the inhibitor of thymidine biosynthesis may independently be about 1.2 times, about 1.5 times, about 2 times, about 2.5 times, about 3, about 4 times, or about 5 times their respective, otherwise preferred, concentration in the composition.

In embodiments wherein the antibiotic composition is used to treat or inhibit a colonisation that is not inside a subject, e.g. a colonisation that is on an object such as a container or a feeding tube, the concentration of at least one of the components of the antibiotic composition may vary from (such as by being greater than) the hereinbefore otherwise preferred concentrations of the components of the composition.

### Other aspects

According to a first aspect of the invention, there is provided a kit, wherein the kit comprises or consists of:
a) a β-lactam antibiotic;
b) a purine nucleoside selected from guanosine and xanthosine, or a combination thereof; and
c) one or more inhibitors of thymidine and/or pyrimidine biosynthesis.

According to another aspect of the invention, there is provided a kit, wherein the kit comprises or consists of:
guanosine or xanthosine; and
an inhibitor of thymidine and/or pyrimidine biosynthesis.

The kit may be for treatment or prevention of a bacterial infection or colonisation.

The components a), b) and c) may be separate compositions. One or more, or all, of the components of a), b) and c) may be in a separate formulation. For example, components a) and b) may be provided as a composition, and component c) as a separate composition. Alternatively, components b) and c) may be provided as a composition, and component a) as a separate composition. Alternatively, components a) and c) may be provided as a composition, and component b) as a separate composition.

Each composition of the kit may be packaged and/or formulated as appropriate for storage. Additionally or alternatively, each composition of the kit may be packaged and/or formulated as appropriate for administration. One or more, or all, of the compositions of the kit may be formulated as a topical, systemic, or localised treatment, or a combination thereof. One or more, or all, of the compositions of the kit may be formulated for enteral (e.g. oral, rectal), parenteral (e.g. intraosseous, intravitreal, intra-articular, intradermal, subcutaneous, intrathecal, epidural, intravenous, intracardiac, intramuscular, or intraperitoneal), buccal, sublingual, transnasal, opthalmic, urogenital/vaginal, or transdermal administration, or for administration by inhalation or by implantation (e.g. of a transdermal implant), or a combination thereof. One or more, or all, of the compositions of the kit may be formulated as cream, ointment, gel, paste, patch, liquid, lotion, powder, tablet, pill, capsule, pastille, lozenge, balm, beverage, emulsion, paste, aerosol, spray, eye or ear drops, pessary, suppository, hydrogel, enema, infusion, impregnated or coated implant. One or more, or all, of the compositions of the kit may be administered indirectly, e.g. *via* catheter or cannula.

The kit may further comprise one or more additional components selected from a syringe, a wipe, an implant, an applicator, a gauze, a container for dispensing (such as a dropper, pipette, spray bottle); or a combination thereof. The kit may comprise at least one carrier, which may be a diluent, such as water, saline or a buffer.

According to another aspect of the invention, there is provided an antibiotic composition or kit according to the invention for use as a medicament.

According to another aspect of the invention, there is provided a method of treatment or prevention of a bacterial infection or colonisation of a subject, the method comprising administration of the antibiotic composition according to the invention to the subject, or the administration of:
a) a β-lactam antibiotic;
b) a purine nucleoside selected from guanosine and xanthosine, or a combination thereof; and
c) an inhibitor of thymidine and/or pyrimidine biosynthesis, to the subject.

The administration/use of a), b) and c) may be concurrently or sequentially. One or more, or all, of the components of a), b) and c) may be administered or used in a separate formulation. In one embodiment, a), b) and c) are administered orally or intravenously, which may be concurrently or separately. In another embodiment, a) and c) are administered orally, which may be concurrently or separately and b) is administered intravenously.

Administration of the composition or components a), b) and c) may be of a therapeutically effective amount. Antibiotic dosing may occur one to three times a day.

According to another aspect of the invention, there is provided a method of treatment or prevention of a bacterial infection or colonisation of a subject, the method comprising administration of the antibiotic composition according to the invention to the subject, or the administration of:
a) guanosine or xanthosine, or a combination thereof; and
b) an inhibitor of thymidine and/or pyrimidine biosynthesis, to the subject.

The administration/use of a) and b) may be concurrently or sequentially. One or both of the components of a) and b) may be administered or used in a separate formulation. In one embodiment, a) and b) are administered orally or intravenously, which may be concurrently or separately. In another embodiment, b) is administered orally, which may be concurrently or separately and a) is administered intravenously. Administration of the composition or components a) and b) may be of a therapeutically effective amount.

According to another aspect of the invention, there is provided a use of the composition or kit according to the invention in the manufacture of a medicament.

The medicament may be for use to treat or prevent a bacterial infection or colonisation of a subject.

According to another aspect of the invention, there is provided a method of reducing resistance to a β-lactam antibiotic in a bacterium, the method comprising the administration of the composition or kit according to the invention.

According to another aspect of the invention, there is provided a method of treatment or prevention of a bacterial biofilm, the bacterial biofilm comprising bacteria, such as antibiotic-resistant bacteria, and the method comprising the administration of the composition according to the invention.

The treatment of the bacterial biofilm may comprise the degradation of the bacterial biofilm, for example by killing or inhibiting a bacterial population of the bacterial biofilm. The biofilm growth may be produced or supported by the targeted bacteria.

According to another aspect of the invention, there is provided a use of the composition or kit according to the invention to kill or inhibit a targeted bacterium, and/or to reduce the number of viable bacteria in an infection, or biofilm, that includes a targeted bacterium.

The use may be *in vitro.* In another embodiment, the use may be *ex vivo.* The use may be for disinfection, for example of an object, surface or liquid.

According to another aspect of the invention, there is provided a method of killing or inhibiting the growth of a targeted bacteria on a surface, object or in a liquid, the method comprising applying the composition or kit according to the invention to the surface, object or in a liquid.

The composition or kit of the invention may be applied by wiping, spraying, injecting, pouring, mixing, and/or submerging the targeted bacterium with or into the composition or kit according to the invention.

### The infection or colonisation / targeted bacterium

The bacteria of the infection or colonisation may be resistant to antibiotic treatment. The bacteria may be resistant to at least one β-lactam antibiotic. The infection or colonisation may be resistant to a penicillin, a cephalosporin, a carbapenem, a variant thereof, and/or a combination thereof. The bacteria may be resistant to methicillin, a variant thereof, and/or a combination thereof. The bacteria may be resistant to oxacillin, cloxacillin, a variant thereof, and/or a combination thereof.

The bacterial infection or colonisation may be caused by at least one strain of antibiotic-resistant bacteria. The bacteria may comprise Gram-positive bacteria. The bacteria may comprise Gram-positive bacteria, Gram-negative bacteria, or a combination thereof. In a preferred embodiment, the bacterium comprises an antibiotic-resistant strain of *Staphylococcus* spp., for example an antibiotic-resistant strain of *Staphylococcus aureus.* The bacteria may comprise methicillin-resistant *Staphylococcus aureus* (MRSA), such as JE2, MW2, COL, or BH1CC.

The bacterial infection or colonisation may comprise a biofilm. The biofilm may be, /or may be suspected to be, inside the subject, for example in a vessel, or alveolar space, of the subject. Alternatively or additionally the biofilm may be, or may be suspected to be, on the subject, for example on the eye, or skin, of the subject. Alternatively or additionally the biofilm may be, or may be suspected to be, in and/or on a surface of an object, for example the biofilm may be in and/or on a catheter, a mask, a ventilator, a feeding tube, a trough, or a food container. The biofilm may be a contaminant of medical equipment.

The bacterial infection or colonisation may comprise, result in, and/or be associated with a condition selected from skin infection, such as dermatitis, cellulitis, or folliculitis; septicaemia; meningitis; gastrointestinal infection, such as dysentery; respiratory infection, such as bronchitis, or chronic obstructive pulmonary disorder; urinary tract infection; sexually transmitted infection; ear infection; nasal infection; throat infection; sinus infection; eye infection, wound infection, and ulcer; or combinations thereof.

In one embodiment, the antibiotic composition or kit of the invention may be administered to treat infected/colonised surfaces, objects, catheters, implants, environmental sources of contamination, water bodies, water/liquid storage vessels, containers (e.g. for food, or for fodder), appliances (such as farming equipment), feeding equipment (e.g. feeding tubes), breathing machines (e.g. intensive care unit breathing equipment), food, beverages, supplements, medicines; or combinations thereof.

### The subject

The subject may be an animal. The subject may be a mammal, a fish, a bird, a reptile, an amphibian, or a domestic animal (e.g. a canine, feline, rodent) and/or a livestock animal (e.g. a bovine, an equine, an ovine, poultry). Preferably, the subject is mammalian. More preferably, the subject is a human.

The subject may be infected or colonised with a targeted bacterium. The subject may not be infected or colonised. The infection or colonisation status of the subject may not be known, for example the subject may be at risk of infection or colonisation, or suspected to be infected or colonised. The composition may be administered to a subject prior to, in concomitance with, or after surgery, optionally wherein the surgery may be unrelated to the infection status of the subject. The composition may be administered to a subject wherein an infection/colonisation has previously been failed (or is believed to have previously been failed) to be treated despite a course of at least one antibiotic, and/or to a subject who has or is at risk of (re-)developing an infection/colonisation recalcitrant to treatment. The subject may be recalcitrant to treatment with one or more conventional antibiotics, such as a beta-lactam antibiotic when used alone.

The composition may be administered to a subject suffering from at least one cancer, and/or a subject suspected to suffer from at least one cancer. The composition may be administered to a subject who is immunocompromised, or believed to be immunocompromised, or at risk of becoming immunocompromised. The composition may be administered to a subject who is afflicted, or believed to be afflicted, by any one or more of skin infection, such as dermatitis, cellulitis, or folliculitis; septicaemia; meningitis; gastrointestinal infection, such as dysentery; respiratory infection, such as bronchitis, or chronic obstructive pulmonary disorder; urinary tract infection; sexually transmitted infection; ear infection; nasal infection; throat infection; sinus infection; eye infection, wound infection, and ulcer.

According to one aspect of the invention, there is provided a composition or kit comprising:
a) up to 960 mg/kg guanosine;
b) oxacillin; and
c) 5-fluorouracil, wherein the oxacillin and 5-fluorouracil are provided in a 1:1 w/w ratio; and optionally
d) a pharmaceutically acceptable carrier.

### Definitions

The expression "antibiotic resistant" and similar expressions, when used in reference to a bacterium such as the targeted bacterium herein, mean bacteria that can survive and/or grow in the presence of a therapeutically relevant amount of at least one β-lactam antibiotic, such as penicillin or methicillin, that would normally inhibit or kill the bacteria. References to resistant infections and resistant biofilms are to be construed accordingly.

The expression "formulated dose" means a formulation that is ready to be administered without requiring prior dissolution and/or dilution of its components.

The expression "pre-formulation" means a formulation that requires dissolution and/or dilution of its components prior to administration.

The terms "treatment" or "therapy" are understood to mean at least a reduction in progression of the disease or a recovery from the disease. In some embodiments, the subject's symptoms of the disease may be fully or partially alleviated. Alternatively or additionally, the terms "treatment" or "therapy" may be understood to mean at least a reduction in the number and/or viability of bacteria in a given colony, or biofilm, or surface, or similar contexts, none of which need necessarily be inside a subject. The treatment effect may be permanent or temporary.

The term "therapeutically effective amount" refers to a medicament amount that achieves treatment, or therapy, as defined hereinabove. For example, a therapeutically effective amount of an antibiotic composition may be an amount of antibiotic composition that kills or inhibits a targeted bacterium, or reduces the viability of the microbes in a colony/biofilm comprising a targeted bacterium. For example, the amount may be curative, or have a therapeutic benefit to a subject.

### General considerations

The skilled person will recognise that features of one aspect or embodiment described herein may be used with any other aspect or embodiment described herein.

As used herein, references to words in the singular, for example when preceded by "a", "an", or "the", are generally to be construed as to mean "one or more" for the purpose of the sentence(s) that follow them, unless the context clearly implies or requires otherwise.

Embodiments of the invention will now be described in more detail, by way of example only, with reference to the accompanying drawings.

### Brief Description of the Tables and Figures

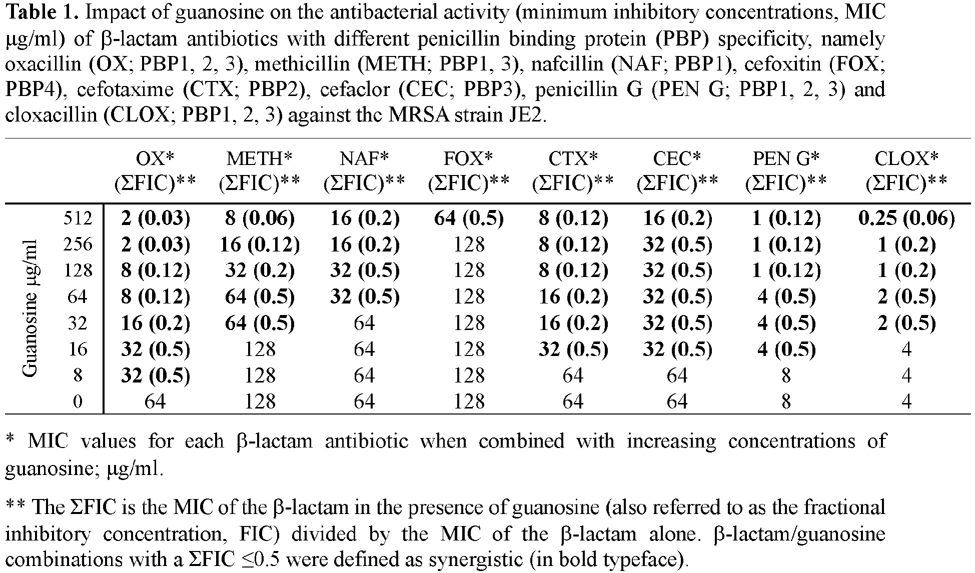

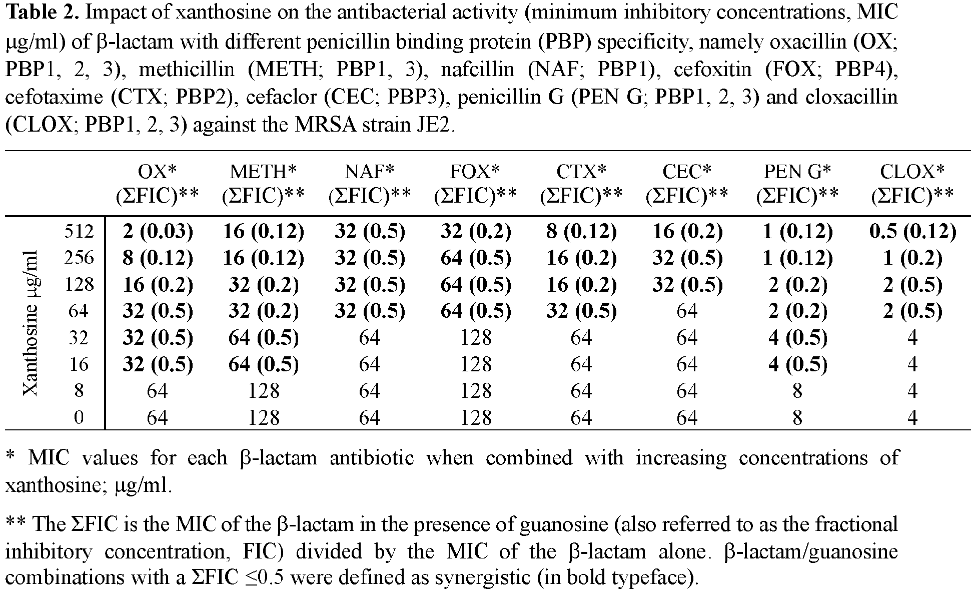

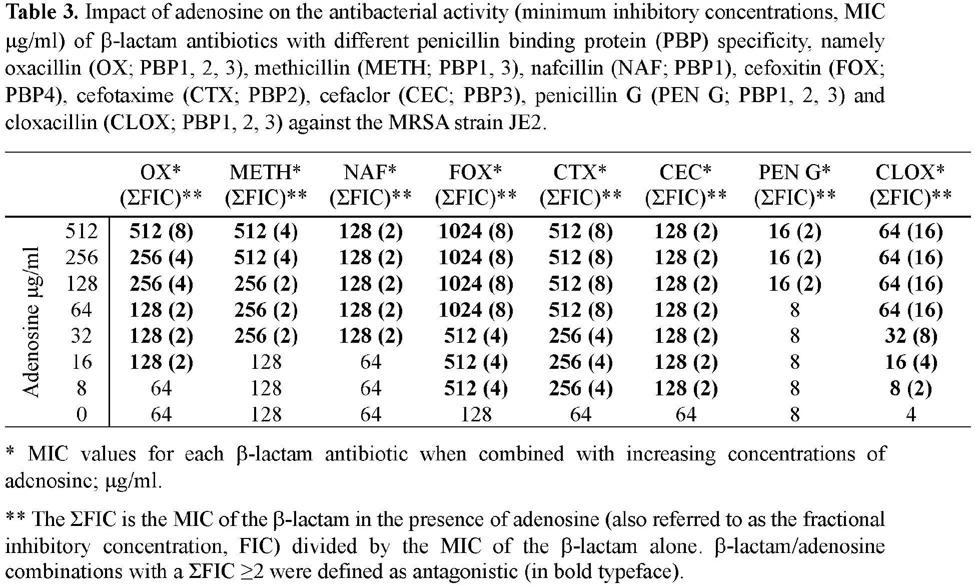

**Table 4. Antibacterial activity (minimum inhibitory concentrations, MIC) and drug synergy (fractional inhibitory concentration indices, ΣFIC) of oxacillin (OX), 5-fluorouracil (5-FU), 5-fluorouridine (5-FUrd), trimethoprim (TMP), sulfamethoxazole (SMX), TPM-SMX in combinations with and without guanosine (Gua, 200 µg/ml), against MRSA strain JE2.**

| Single antibiotic | MIC (µg/ml)* | | |
|---|---|---|---|
| OX | 64 | | |
| 5-FU | 32 | | |
| 5-FUrd | 0.06 | | |
| TMP | 1 | | |
| SMX | 128-256 | | |
| TMP-SMX | 0.25 | | |

| Single + Gua antibiotic | MIC (µg/ml)** | | ΣFIC*** |
|---|---|---|---|
| OX/Gua | 2-4 | | **0.03** |
| 5-FU/Gua | 16 | | **0.5** |
| 5-FUrd/Gua | 0.03 | | **0.5** |
| TMP/Gua | 1 | | *1* |
| SMX/Gua | 128 | | *1* |
| TMP-SMX/Gua | 0.5 | | *1* |

| Double antibiotic combinations | MICs (µg/ml) | | ΣFIC |
|---|---|---|---|
| OX/5-FU | OX 8 | 5-FU 8 | **0.25** |
| OX/5-FUrd | OX 0.25 | 5-FUrd 0.012 | **0.2** |
| OX/TMP | OX 0.25 | TMP 0.25 | **0.25** |
| OX/SMX | OX 16 | SMX 64 | *0.75* |
| OX/TMP-SMX | OX 32 | TMP-SMX 0.12 | *1* |

| Double antibiotic + Gua combinations | MICs (µg/ml)**** | | ΣFIC |
|---|---|---|---|
| OX/5-FU/Gua | OX 0.5 | 5-FU 0.5 | *0.016* |
| OX/5-FUrd/Gua | OX <0.06 | 5-FUrd <0.003 | <*0.05* |
| OX/TMP/Gua | OX 0.25 | TMP 0.12 | *0.12* |
| OX/SMX/Gua | OX 0.5 | SMX 8 | *0.03* |
| OX/TMP-SMX/Gua | OX 1 | TMP-SMX 0.03 | *0.12* |

| | | | |
|---|---|---|---|
| * MIC, minimum inhibitory concentration; µg/ml. ** MIC of the antibiotic in the presence of Gua or another antibiotic; µg/ml. *** FIC indices (ΣFIC) for antibiotic/Gua, antibiotic/antibiotic and antibiotic/antibiotic + Gua combinations. The fractional inhibitory concentration (FIC) is the MIC of the antibiotic in the presence of Gua or another antibiotic divided by the MIC of the antibiotic alone); µg/ml. The ΣFIC = FIC A + FIC B, where FIC A is the MIC of the first antibiotic in combination with Gua or another antibiotic/MIC of the first antibiotic alone, and FIC B is the MIC of the second antibiotic in combination with Gua or another antibiotic/MIC of the second antibiotic alone. The combination is considered synergistic when the ΣFIC is ≤0.5 (in bold typeface), indifferent when the ΣFIC is >0.5 to <2 (in italics). * * * * When two antibiotics were used in combination with each other in the presence of Gua, the MIC of each antibiotic in the presence of both the second antibiotic and Gua is indicated. | | | |

### Figures

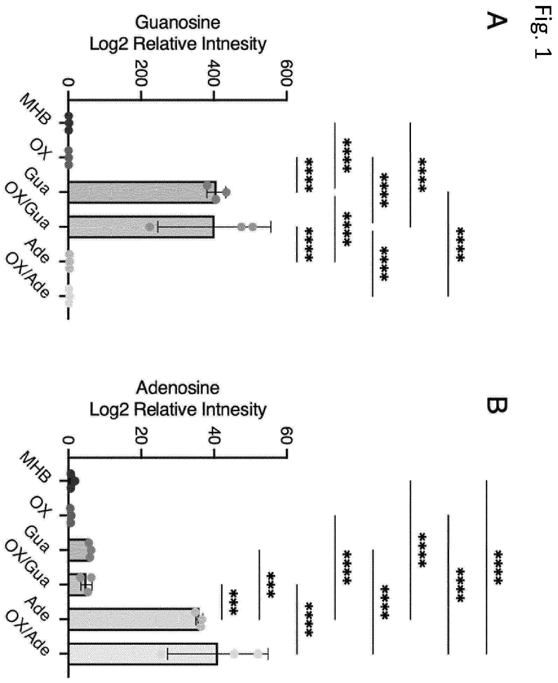

**Fig. 1****. Impact of exogenous guanosine or adenosine alone and in combination with oxacillin on intracellular guanosine, adenosine, asparagine and glutamine levels in MRSA strain JE2.** (A) Intracellular guanosine and (B) intracellular adenosine were quantified in cells grown in MHB, MHB oxacillin (OX, 1 µg/ml), MHB guanosine (Gua, 200 µg/ml), MHB OX/Gua, MHB adenosine (Ade, 200 µg/ml) and MHB OX/Ade. Data presented are the average of three biological replicates plotted using GraphPad Prism V9. Asterisks indicate statistically significant difference according to using a one-way ANOVA test. p-values * p<0.05, ** p<0.005, *** p<0.001 and **** p<0.0001 are indicated.

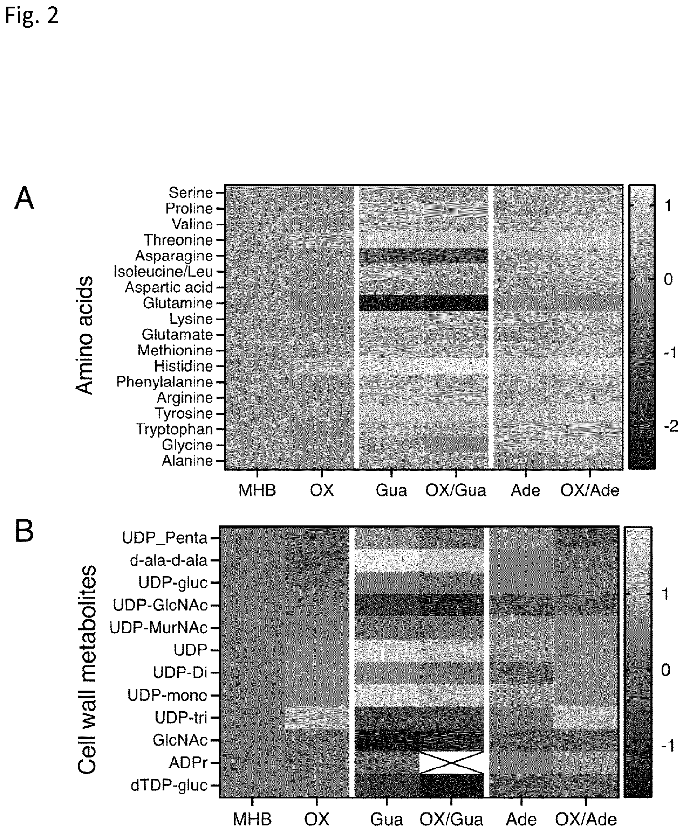

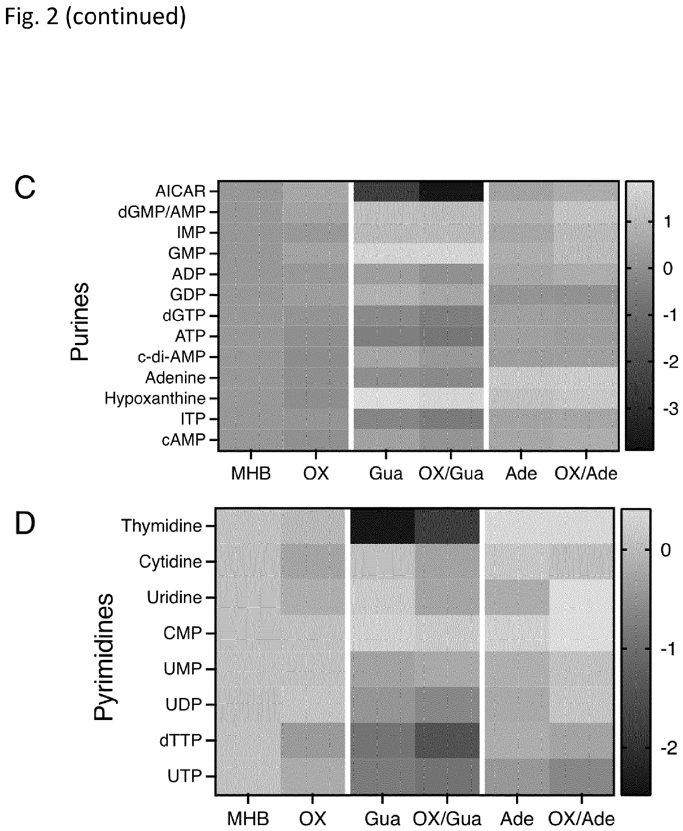

**Fig. 2****.** Heatmap comparison of (A) amino acids, (B) cell wall metabolites, (C) purines and (D) pyrimidines in JE2 grown in MHB and MHB supplemented with guanosine (Gua, 200 µg/ml) or adenosine (Ade, 200 µg/ml) with or without oxacillin (OX, 1 µg/ml). The data presented are the average of three biological replicates analysed using GraphPad Prism V9. Individual metabolite levels that were significantly different were identified using a one-way ANOVA analysis.

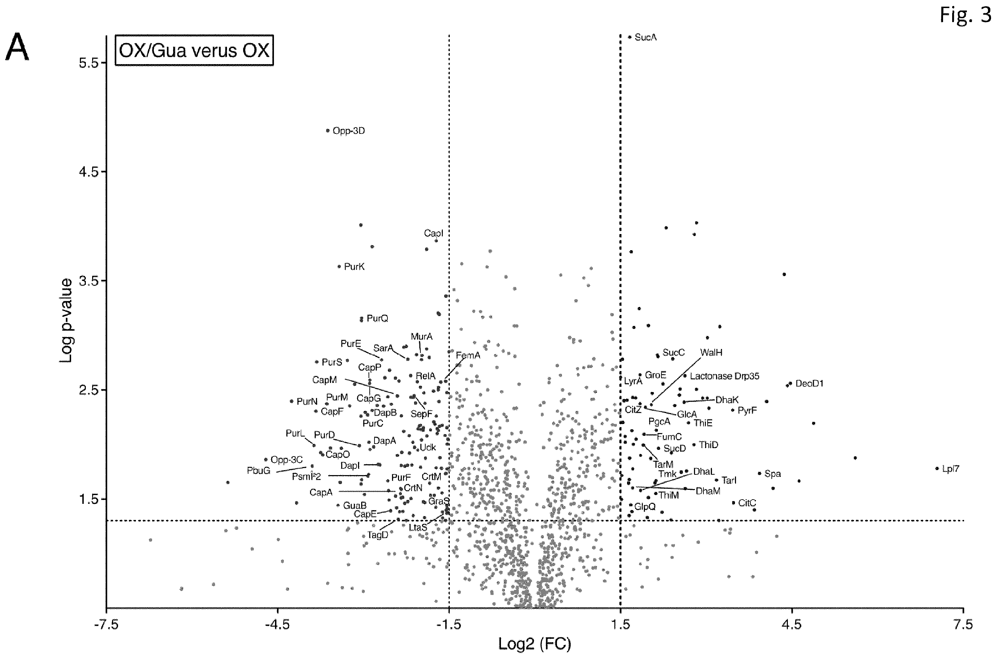

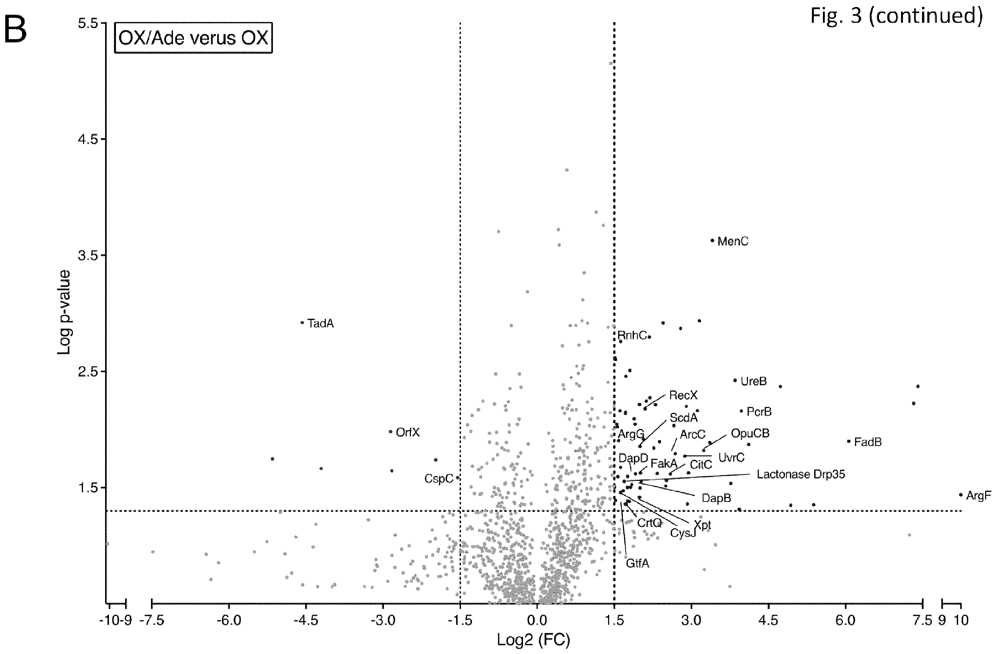

**Fig. 3****.** Comparative proteomic analysis of JE2 grown in MHB supplemented with (A) oxacillin (1 µg/ml) and guanosine (200 µg/ml) (OX/Gua) versus OX alone or (B) OX and adenosine (200 µg/ml) (OX/Ade) versus OX alone. Volcano plots of intracellular proteins were generated using GraphPad Prism V9. The horizontal dotted line indicates the cutoff for proteins with significantly altered abundance (P ≤ 0.05) and vertical dotted lines indicate proteins with significantly reduced or increased abundance (≥1.5 log difference) in OX/Gua or Ade/Gua versus OX alone.

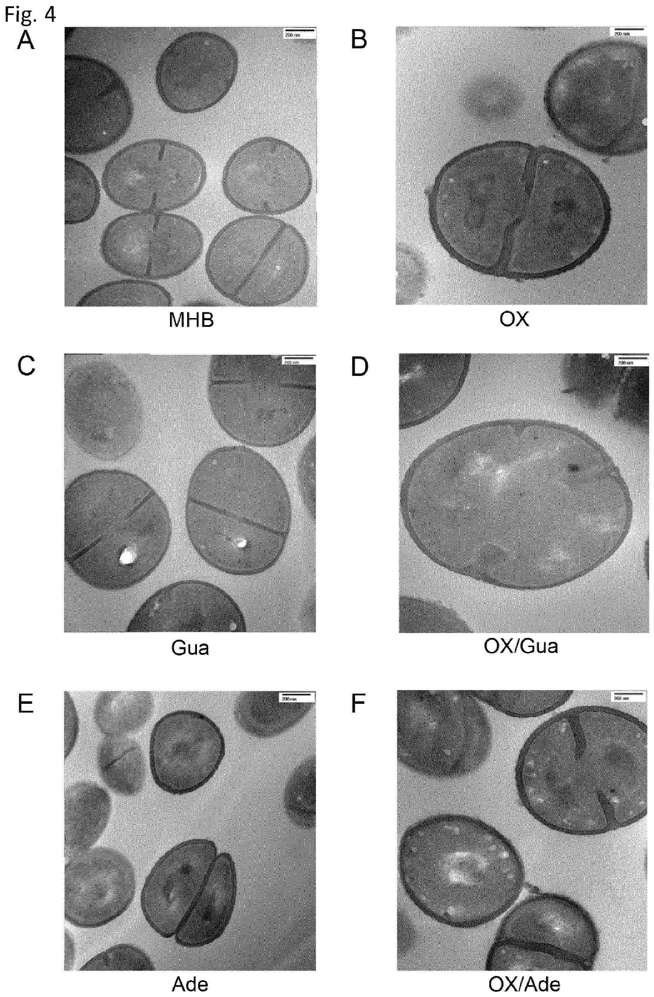

**Fig. 4****.** Representative transmission electron microscopy images of MRSA cells grown in (A) MHB, (B) MHB oxacillin (OX, 1 µg/ml), (C) MHB guanosine (Gua, 200 µg/ml) (D) MHB OX/Gua (E) MHB adenosine (Ade, 200 µg/ml) and (F) MHB OX/Ade. Black scale bars = 200 nm.

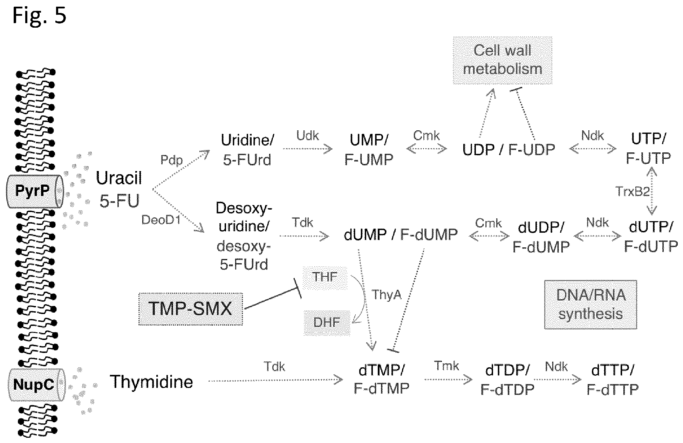

**Fig. 5****. Overview of thymidine, uracil and 5-fluorouracil (5-FU) transport and metabolism including enzymes and intermediates implicated in susceptibility to 5-FU and trimethoprim-sulfamethoxazole (TMP-SMX).** The uracil permease PyrP (also known as UraA) transports uracil and presumably 5-FU. Uracil is converted to uridine and desoxy-uridine by Pdp and DeoD1, respectively, before being fluxed to UMP/dUMP, UDP/dUTP and UTP/dUTP. dUMP is converted to dTMP by thymidylate synthase (ThyA) in a reaction requiring tetrahydrofolic acid (THF) as a co-factor, which is converted to dihydrofolic acid (DHF). UDP is an important intermediate in cell wall metabolism, which is adversely affected by the antimetabolite 5-F-UDP. 5-FU exposure leads to the buildup of F-UMP and F-TMP, which interferes with the normal incorporation of UMP and TMP into DNA and RNA, respectively. TMP-SMX inhibits two enzymatic reactions in the folate pathway thereby interfering with the synthesis of THF required for ThyA activity. Exogenous thymidine is transported by NupC, bypassing the phenotypic impact of TMP-SMX treatment on ThyA activity.

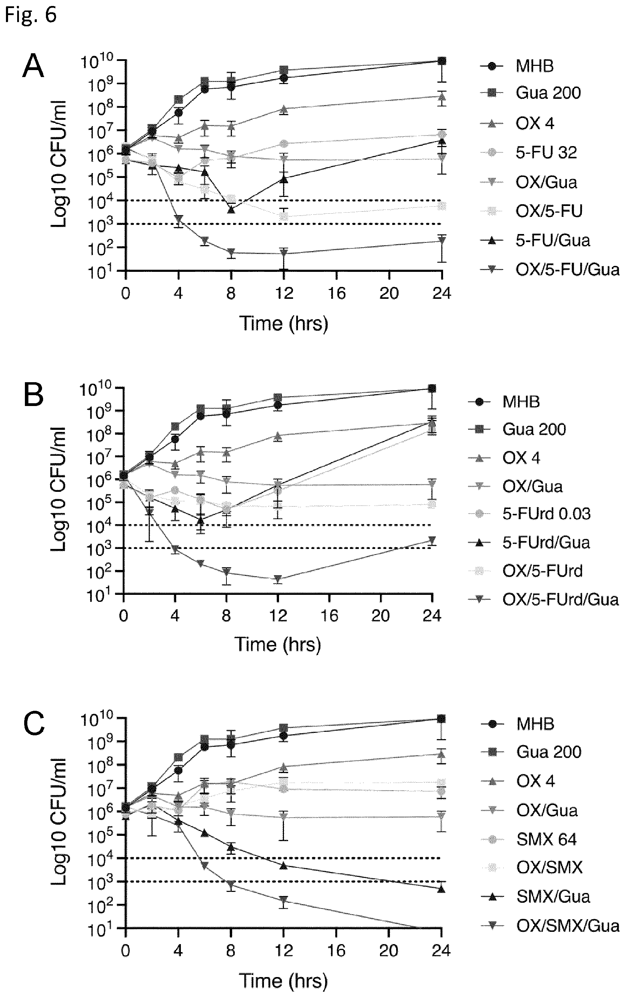

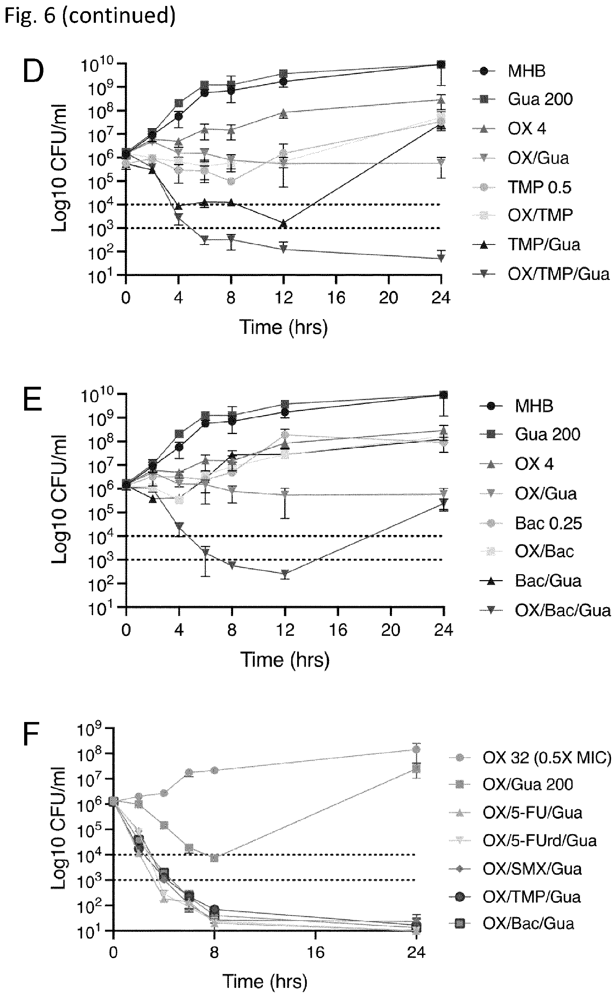

**Fig. 6****.** MRSA killing by 0.5× minimum inhibitory concentrations of 5-fluorouracil (5-FU) 32 µg/ml (A), 5-fluorouridine (5-Furd) 0.03 µg/ml (B), sulfamethoxazole (SMX) 64 µg/ml (C), trimethoprim (TMP) 0.5 µg/ml (D), bacitracin (Bac) 0.25 mg/ml (E) alone and in combination with guanosine (Gua) 200 mg/ml and/or oxacillin (OX) 4 mg/ml; 0.0625× MIC (A-E) or OX 32 µg/ml; 0.5× MIC (F). Exponential phase cultures were inoculated into MHB 2% NaCl at a starting cell density of approximately 1×10⁶ CFU/ml, with or without Gua and antibiotics as indicated, and CFUs enumerated after 0, 2, 4, 6, 8, 12, and 24 h. The data presented are the average of three independent experiments and standard deviations are shown.

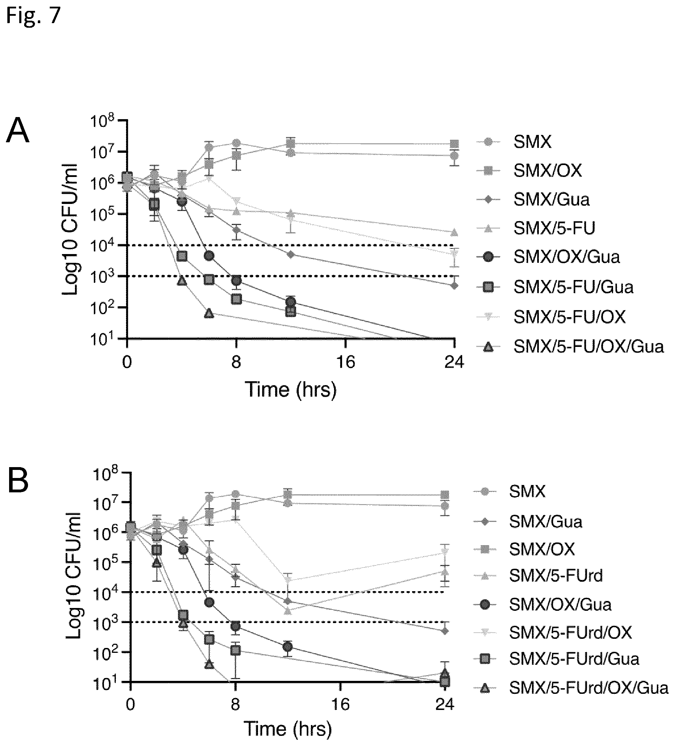

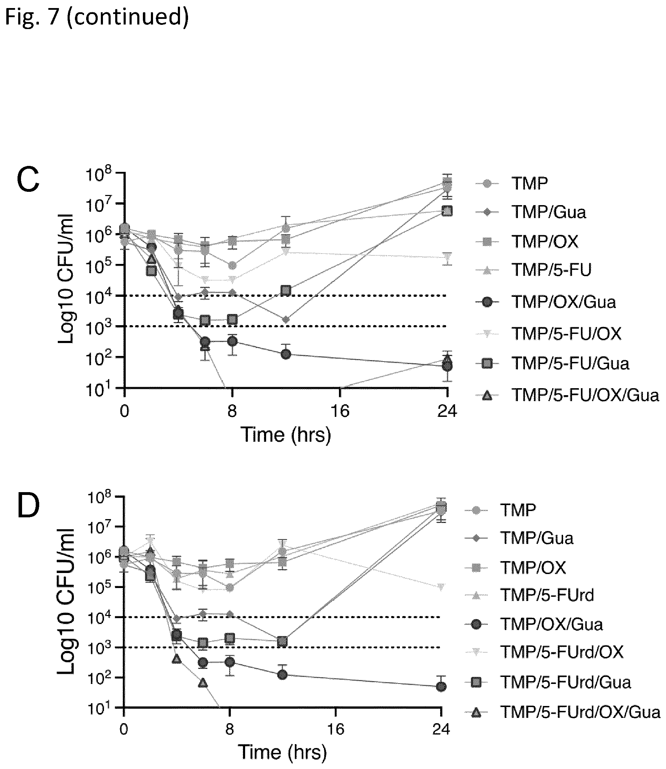

**Fig. 7****.** MRSA killing by 0.5× minimum inhibitory concentrations of sulfamethoxazole (SMX; 64 µg/ml), 5-fluorouracil (5-FU; 32 µg/ml), 5-fluorouridine (5-FUrd; 0.03 µg/ml) and trimethoprim (TMP; 0.5 µg/ml) alone and in various combinations with guanosine (Gua) 200 µg/ml and/or oxacillin (OX; 4 µg/ml; 0.0625× MIC). (A) SMX along and in combinations with OX, 5-FU and Gua. (B) SMX alone and in combinations with OX, 5-FUrd and Gua. (C) TMP alone and in combinations with OX, 5-FU and Gua. (D) TMP alone and in combinations of with OX, 5-FUrd and Gua. Exponential phase cultures were inoculated into MHB 2% NaCl at a starting cell density of approximately 1×10⁶ CFU/ml, with or without antibiotics and Gua as indicated, and CFUs enumerated after 0, 2, 4, 6, 8, 12, and 24 h. The data presented are the average of three independent experiments and standard deviations are shown.

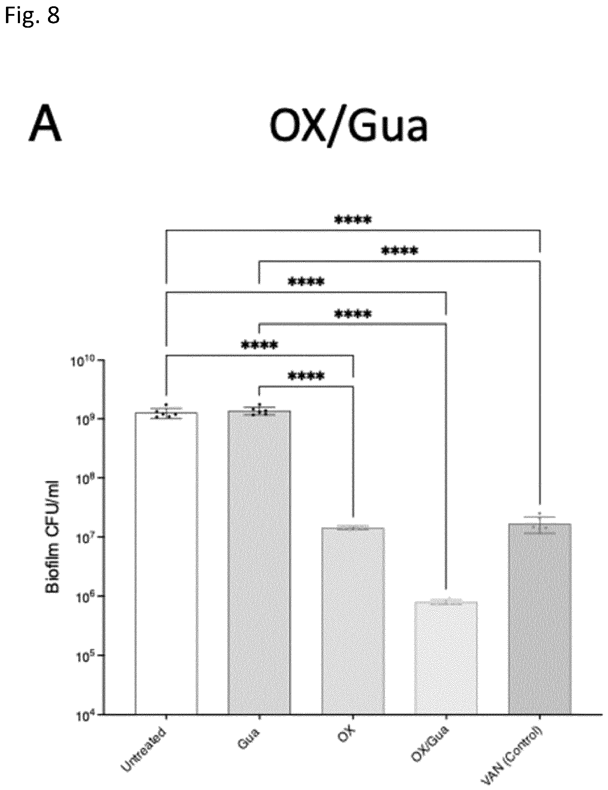

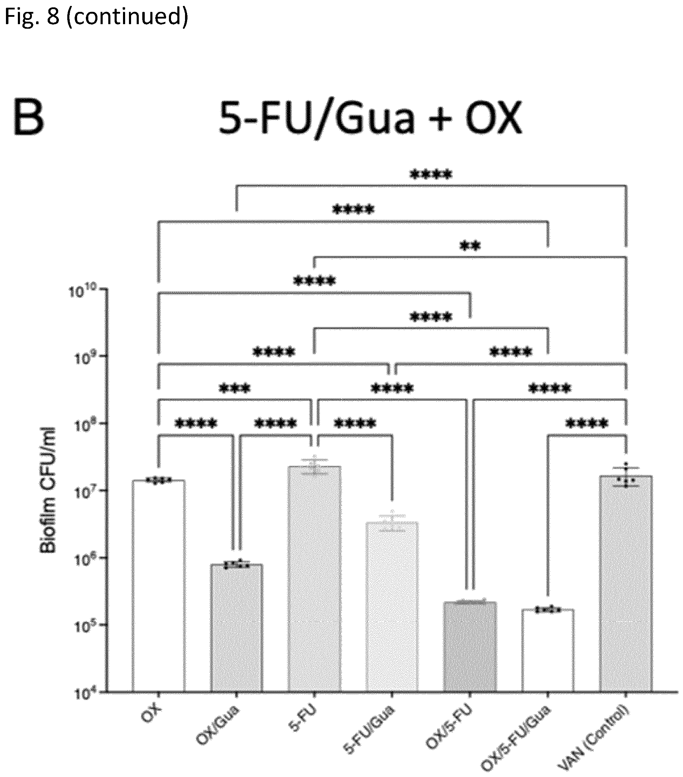

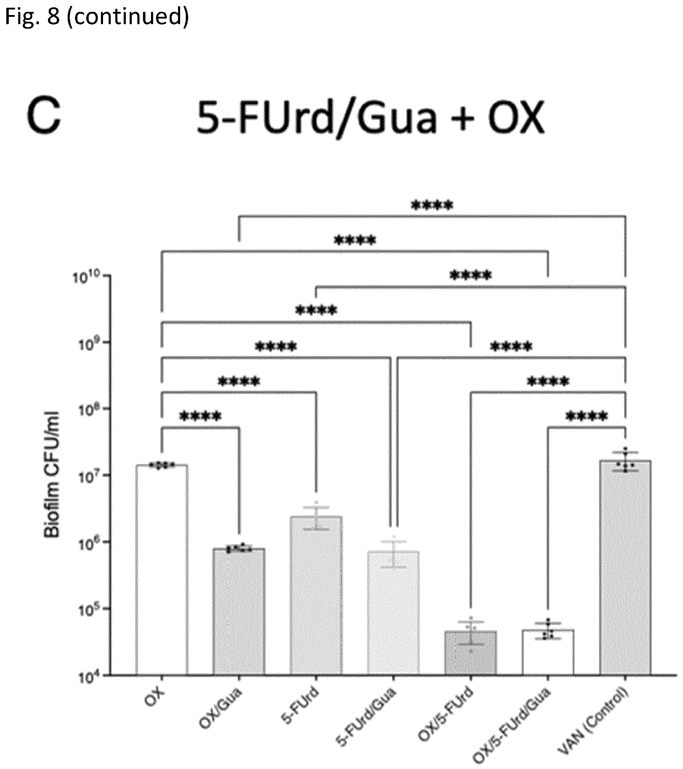

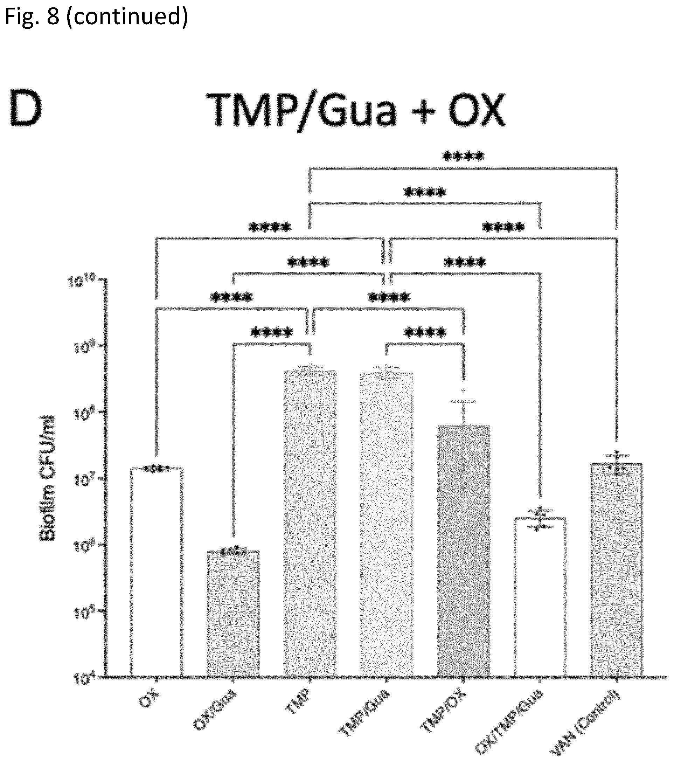

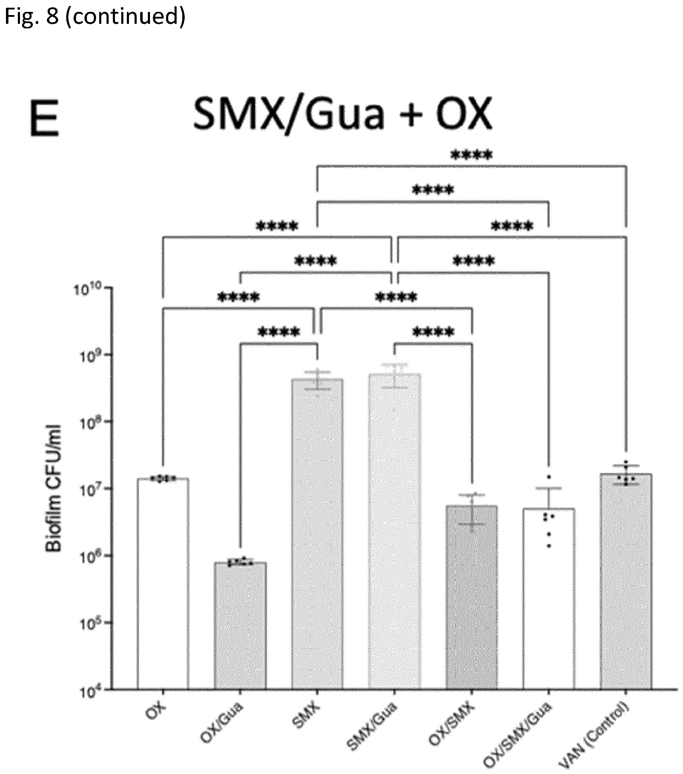

**Fig. 8****. Oxacillin, 5-fluorouracil, 5-fluorouridine and guanosine combinations significantly eradicate MRSA JE2 biofilms.** Comparison of untreated JE2 biofilm with eradication by (A) guanosine (Gua, 200 µg/ml), oxacillin (OX, 200 µg/ml), OX/Gua, (B) 5-fluorouracil (5-FU, 68 µg/ml), 5-FU/Gua, OX/5-FU, OX/5-FU/Gua, (C) 5-fluorouridine (5-FUrd, 200 µg/ml), 5-Furd/Gua, OX/5-FUrd, OX/5-FUrd/Gua, (D) trimethoprim (TMP, 4 µg/ml), TMP/Gua, OX/TMP, OX/TMP/Gua, and (E) sulfamethoxazole (SMX, 78 µg/ml), SMX/Gua, OX/SMX, OX/SMX/Gua. Vancomycin (VAN, 50 µg/ml) was used as a control for all experiments. Biofilms were grown in tryptic soya broth 0.5% glucose in 24-well plates for 24 h followed by 24 h treatment with antibiotics. Biofilm viability after antibiotic treatment was measured by enumerating colony forming units (CFU/ml). The data presented are the average of three biological replicates plotted using GraphPad Prism V9. Asterisks indicate statistically significant differences according to One-way Anova analysis (**** p<0.05). Error bars indicate standard deviation.

### Results

**Purine adjuvants downregulate thymidine biosynthesis revealing a new therapeutic target.** To further investigate the therapeutic potential and mechanism of action of purine nucleosides, we first compared the anti-MRSA activity of several b-lactam antibiotics in combination with guanosine (Gua), xanthosine (Xan) and adenosine (Ade) (Tables 1 and 2). The most significant synergy was measured between Gua or Xan and oxacillin (OX) (fractional inhibitory concentration index (ΣFIC) = 0.03, followed by cloxacillin and methicillin (Tables 1 and 2; ΣFIC's = 0.06). As noted previously (1), Ade significantly antagonised the anti-MRSA activity of all b-lactams tested (Table 3; ΣFIC's = 2-16).

Comparison of intracellular metabolites in MHB cultures supplemented with these nucleosides alone or with OX (1 µg/ml) revealed significant accumulation of Gua (Fig. 1A) or Ade (Fig. 1B). More broadly, nucleoside-treated JE2 cultures exhibited pleiotropic effects (Fig. 2A-D), including significant depletion of glutamine and asparagine in MHB Gua (Fig. 2A). Glutamine is an important NH₄⁺ donor for the amidation of iso-D-glutamate in the peptidoglycan stem pentapeptide (3), suggesting that glutamine depletion may interfere with cell wall synthesis. Asparagine is an important intermediate in purine metabolism contributing to MRSA survival during bone infection (4). Accumulation of Gua was accompanied by decreased levels of the cell wall precursors GlcNAc, UDP-GlcNAc and UDP-tripeptide (UDP-tri), whereas D-ala-D-ala, UDP and UDP-monopeptide (UDP-mono) were increased (Fig. 2B). Exposure to Ade had a less significant effect on cell wall metabolites; only UDP-tri was increased in MHB OX/Ade (Fig. 2B). Notably, the purine biosynthetic intermediate AICAR and the pyrimidine nucleotide thymidine were significantly downregulated in Gua-treated cells (Fig. 2C,D). The correlation between downregulation of thymidine and reduced β-lactam resistance in MRSA indicated that this metabolite could be key in developing novel therapeutic interventions in resensitising MRSA to antibiotic treatment.

Comparative whole cell proteomics further revealed dramatically different impacts of Gua versus Ade exposure (Fig. 3) on the MRSA proteome with or without OX. In OX/Gua versus OX alone, 248 proteins showed significantly altered abundance (86 upregulated, 162 downregulated) (Fig. 3A). In contrast only 82 proteins exhibited altered abundance in OX/Ade versus OX alone, and the majority of these (75 proteins) were upregulated (Fig. 3B). Consistent with depleted AICAR levels (Fig. 2C,D), multiple proteins in the *de novo* purine biosynthesis (PurQESNMCDFL) and salvage (PbuG, GuaB) pathways displayed lower abundances in OX/Gua versus OX (Fig. 4A).

Levels of RelA, which synthesises the stringent response alarmone (p)ppGpp required for OX resistance (5) were also lower. Numerous proteins related to staphyloxanthin synthesis (CrtNM), cell wall biosynthesis (MurA, FemA, DapABI), capsule assembly (CapAEGMOP) and teichoic acid synthesis (LtaS, TagB) were also downregulated in Gua/OX indicating perturbations in cell envelope structure and the ability to cope with oxidative stress. Pathways upregulated in OX/Gua included pyrimidine biosynthesis (Tmk, PyrF), wall teichoic acid degradation/synthesis (GlpQ, TarM, TarI), peptidoglycan cleavage (LyrA), lipoteichoic acid synthesis (PgcA), TCA cycle (SucCD, FumC, CitCZ) and stress response (DhaKLM, GroE).

Proteins upregulated in OX/Ade versus OX (Fig. 3B) included DapBD and CrtQ from the cell wall and staphyloxanthin biosynthesis pathways that were upregulated in Gua/OX perhaps indicating that Gua and Ade have different effects on cell envelope structure. Consistent with previous confocal microscopy analysis (1), transmission electron microscopy visualisation showed that JE2 MRSA cells exposed to OX/Gua had aberrant septa, and exhibited significantly increased cell diameters and thinner cell walls compared to cells grown in OX/Ade (Fig. 4). Taken together, these data reveal the pleiotropic effects of MRSA exposure to OX/Gua and identify thymidine depletion as a potentially important element of the mechanism of action of this therapeutic combination.

**Anti-MRSA activity of oxacillin combined with 5-fluorouracil and antifolates is significantly increased by guanosine.** Downregulation of thymidine levels by OX/Gua raised the hypothesis that purine adjuvants may also potentiate the activity of antibiotics that interfere with pyrimidine and/or thymidine biosynthesis. The pyrimidine analogue drug 5-fluorouracil (5-FU) disrupts pyrimidine metabolism and has previously been reported to cause "thymidine-less death" in bacteria (6). Exogenous 5-FU is taken up and converted into fluorinated analogues of uridine/deoxyuridine; 5-fluorouridine/5-fluorodeoxyuridine (5-FUrd/deoxy 5-FUrd) (6-8) (Fig. 5). 5-FU and 5-FUrd were reported to have antitumour properties in humans (9, 10). Trimethoprim (TMP) and sulfamethoxazole (SMX) block the activity of dihydrofolate reductase and dihydropteroate synthase respectively, from the pathway leading to production of tetrahydrofolate, which is a co-factor for thymidylate synthase (ThyA) (Fig. 5), and purine biosynthetic enzymes PurN and PurH (11). The combination anti-folate drug TMP-SMX (1:5), a formulation also known as Bactrim, is commonly used to treat *S*. *aureus* and MRSA infections (12).

Using checkerboard assays, synergy was detected between Gua and 5-FU or 5-FUrd against JE2, but not with TMP, SMX or TMP-SMX (Table 4). Combinations of OX with 5-FU, 5-FUrd and TMP were also synergistic (Table 4). Gua (200 µg/ml) potentiated the activity of all these antibiotic combinations (as evidenced by lower ΣFIC values) including OX/SMX and OX/TMP-SMX, which alone were not synergistic (Table 4).

Using kill curve assays to investigate potential bactericidal activity, significant synergy was measured between 5-FU, 5-FUrd, SMX and TMP (0.5× MICs) and Gua as defined by a >2 log reduction in the number of JE2 colony forming units (CFUs)/ml (Fig. 6A-D). No synergy was detected for TMP-SMX/Gua in kill curve assays (Fig. 6E). Notably bactericidal activity (> 3 log CFU reduction) was achieved when low dose OX (4 µg/ml; 0.0625× MIC) was added to these antibiotic/Gua combinations (Fig. 6A-E). Further increasing the OX concentration to 32 µg/ml (0.5× MIC) achieved a near eradication of JE2 after 24 hours for all 5-FU, 5-FUrd, SMX, TMP and TMP-SMX combinations (Fig. 6F).

Finally, the ability of Gua to potentiate the anti-MRSA activity of combinations of the anti-folates SMX or TMP and the pyrimidine analogues 5-FU or 5-FUrd was measured (Fig. 7A-D). No significant synergy was measured for SMX/5-FU, SMX/5-FUrd, TMP/5-FU or TMP/5-FUrd combinations, even when low dose OX (4 µg/ml) was included (Fig. 7A,(b)). Strikingly, the addition of Gua to the SMX/5-FU and SMX/5-FUrd combinations was bactericidal and achieved a near eradication of JE2 after 24 hours (Fig. 7A,(b)). Gua also potentiated the activity of TMP/5-FU and TMP/5-FUrd, without achieving bactericidal activity and with JE2 CFU's recovering after 24h (Fig. 7C,D). The most significant bactericidal activity was measured when Gua was included in triple SMX/5-FUrd/OX, TMP/5-FU/OX, TMP/5-FUrd/OX and SMX/5-FU/OX combinations, which achieved bactericidal activity and near eradication of JE2 after 8-16 hr (Fig. 7A-D). Taken together, these findings reveal that combinations of the purine adjuvant Gua with these anti-folate and pyrimidine analogue drugs have significant therapeutic potential to enhance the treatment of MRSA infections, particularly if OX (even at sub-MICs) is also administered.

**Eradication of MRSA biofilms by antibiotic/guanosine combinations.** Combinations of OX, 5-FU, 5-FUrd, TMP and SMX with or without Gua also eradicated preformed JE2 biofilms. In these experiments, antibiotic concentrations equal to or less than the Cₘₐₓ in human serum were used i.e. OX 200 µg/ml (13, 14), SMX 78 µg/ml (15), TMP 4 µg/ml (15), 5-FU 68 µg/ml (16), 5-FUrd 200 µg/ml (17) and vancomycin (VAN, control) 50 µg/ml (18) were used in various combinations with 200 µg/ml Gua, for which an upper toxicity limit *in vivo* has not been reported (19).

OX, 5-FU or 5-FUrd significantly eradicated biofilm particularly when combined with Gua (Fig. 8A-C). TMP and SMX alone or in combination with Gua were ineffective for biofilm eradication (Fig. 8D,E). Combinations of OX, 5-FU or 5-FUrd and to a lesser extent TMP or SMX achieved 2-4 log reductions (Fig. 8). Although Gua did not potentiate biofilm eradication by OX/5-FU or OX/5-FUrd (Fig. 8), the 4-5 log reduction of biofilm CFUs by OX/5-FUrd and OX/5-FUrd/Gua was significantly better than the 2-log reduced achieved by VAN (Fig. 8) and the previously reported 1-3 log reductions achieved by single antimicrobial agents (20). Moreover this level of biofilm eradication is regarded as disinfection and comparable to other antimicrobial combinations reported to have effective anti-biofilm activity (20-22).

### Methods

**Comparative metabolomic analysis.** Intracellular metabolite analysis was performed as described previously (23, 24). 250 ml flasks containing 25 ml MHB (with or without 1 µg/ml OX, 200 µg/ml Gua or 200 µg/ml Ade) were inoculated from 5ml overnight cultures at a starting OD₆₀₀=0.06 and grown for 4-5 h. Culture volumes corresponding to OD₆₀₀=10 were harvested and rapidly filtered through a 0.45 µm membrane (Millipore). The cells on the membrane were washed twice with 5 ml cold saline and immediately quenched in ice-cold 60% ethanol containing 2 µM Br-ATP as an internal control. The cells were mechanically disrupted using a bead homogenizer set to oscillate for 3 × 30 s cycles at 6800 rpm with a 10 s pause between each cycle. Cell debris was separated by centrifugation at 12,000 rpm. Supernatants containing intracellular metabolites were lyophilized and stored at -80°C. These samples were reconstituted in 100 µl of 50% MeOH. LC-MS/MS analysis was carried out as described previously (25).

**Comparative proteomic analysis by LC-MS/MS.** 250 ml flasks containing 25 ml MHB (with or without 1 µg/ml OX, 200 µg/ml Gua or 200 µg/ml Ade) were inoculated from 5ml overnight cultures at a starting OD₆₀₀=0.06 and grown for 4-5 h. Culture volumes corresponding to OD₆₀₀=12 were placed on ice and harvested. Cells were washed twice with ice-cold PBS, before being resuspended in 100 ml TFA and incubated for 15 minutes at 95 °C. Thereafter 1ml of 2M TrisBase was added, followed by 110 µl 100 mM Tris(2-carboxyethyl)phosphine and 110 µl 400mM 2-Chloroacetamide and incubation at 95 °C for a further 5 minutes (26). The total protein concentration was measured using the Pierce BCA Assay Kit (Promega) and the samples were adjusted to 0.1 mg/ml protein concentration before being incubated with 0.001 mg/ml trypsin for 20 hours at 37 °C in a thermomixer at 600rpm. This was then followed with 10mM DTT treatment at 56°C for 30 mins to reduce the peptides, which was subjected to alkylation with 50 mM iodoacetamide for 20 mins in the dark. The resulting peptides were subjected to identification and quantification using LC-MS/MS analysis established previously (27). Briefly, the peptides were cleaned using PepClean C18 spin columns, and resuspended in 2% acetonitrile (ACN) and 0.1% formic acid (FA) buffer. Five hundred ng of each sample was loaded onto trap columns (Acclaim PepMap) as previously described (27) with a step gradient of 4-25% solvent B (0.1% FA in 80 % ACN) from 10-100 min and 25-45% solvent B for 100-130 min, and at 50°C with a total of 155 min total run. The eluted samples were analysed in mass spectrometer in a data-dependent acquisition mode as previously described (27). MS/MS data analysis was done using PEAKS Studio X+ software against an in-house database created from predicted *S*. *aureus* USA300 FPR3757 proteins. The significance threshold of the ion score was calculated based on a false discovery rate of ≤1%. Statistical analysis using ANOVA and the Benjamini-Hochberg (BH) method was used to adjust P values for multiple-testing using the false discovery rate. The adjusted p≤ 0.05 was considered significant (27).

**Transmission electron microscopy (TEM) and cell morphology analysis.** Triplicate JE2 Mueller Hinton broth cultures supplemented with guanosine (200 µg/ml), adenosine (200 µg/ml) and oxacillin (1 µg/ml) as indicated cultures were grown for 5 hours at 35 °C with shaking at 200rpm before being pelleted, resuspended in PBS and adjusted to OD₆₀₀=1 in PBS. Cells were again pelleted and resuspended/fixed with 0.2M sodium cacodylate buffer pH 7.2 and subsequently 1% osmium tetroxide in 0.1M sodium cacodylate buffer pH 7.2. Fixed cells were dehydrated by serial immersion for 15 m each in 30%, 50%, 70%, 90% and 100% ethanol before thin sections were prepared. Images were acquired using a Hitachi H7500 Transmission Electron Microscope. Cell wall thickness (at 3 locations per cell) and cell diameter were measured for non-dividing cells using ImageJ software for 30 cells (10 per biological replicate).

**Antibiotic minimum inhibitory concentration (MIC) measurements and synergy/checkerboard assays.** MIC measurements by broth microdilutions were performed in accordance with CLSI methods for dilution susceptibility testing of staphylococci (28) with modifications as follows; guanosine, xanthosine or adenosine were supplemented into culture media at a final concentration of 200 µg/ml. Strains were first grown at 37°C on MHA 2% NaCl for 24 h and 5-10 colonies were resuspended in 0.85% saline before being adjusted to 0.5 McFarland standard (OD₆₀₀=0.1). The cell suspensions were then diluted 1:20 in PBS and 10 µl used to inoculate 100 µl media (MHB 2% NaCl/ MHB 2% NaCl with 200 µg/ml guanosine) containing serially diluted antibiotics (oxacillin, 5-fluorouracil, 5-fluorouridine, sulfamethoxazole, trimethoprim) as indicated. The plates were incubated at 37 °C for 24 h and MIC values were recorded as the lowest antibiotic concentration where no growth was observed. Checkerboard/synergy assays performed as previously described, utilised 96 well plates in which one antibiotic/adjuvant was serially diluted vertically and the second antibiotic was serially diluted horizontally.

**Antibiotic kill curves.** Antibiotic kill curves were performed according to CLSI guidelines as previously described (1). Briefly, overnight cultures grown in MHB 2% NaCl were diluted 1:100 and grown for 3 h before being inoculated into 25 ml of MHB NaCl with or without antibiotics as indicated in 250 ml flasks at a starting cell density of approximately 1×10⁶ CFU/ml. The flasks were incubated at 35°C with shaking at 200 rpm and CFU were enumerated on tryptone soya agar plates after 0, 2, 4, 6, 8, 12, and 24 h.

**Biofilm eradication assays.** Biofilm eradication assays were performed as described previously (29). Briefly, biofilms were grown in 24-well tissue culture treated plates at 37 °C for 24 hours before being carefully washed twice with PBS. BHI supplemented with antibiotics as indicated was then added to the biofilm wells and the biofilms incubated for a further 24 h at 37 °C. The biofilms were dispersed by scraping, serially diluted and CFUs enumerated on TSA plates. The antibiotic concentrations used in these experiments was equal to or less than the Cₘₐₓ in humans as follows: oxacillin 200 µg/ml (13), sulfamethoxazole 78 µg/ml, trimethoprim 4 µg/ml (15), 5-fluorouracil 68 µg/ml (16), 5-fluorouridine 200 µg/ml (17) and vancomycin 50 µg/ml (18). An upper *in vivo* toxicity limit for guanosine, which was used at 200 µg/ml, has not been reported (19).

All references are incorporated herein by reference.

### References

1. Nolan AC, Zeden MS, Kviatkovski I, Campbell C, Urwin L, Corrigan RM, Gründling A, O'Gara JP. 2023. Purine Nucleosides Interfere with c-di-AMP Levels and Act as Adjuvants To Re-Sensitize MRSA To β-Lactam Antibiotics. mBio 14:e02478-22.
2. Douglas EJA, Wulandari SW, Lovell SD, Laabei M. 2023. Novel antimicrobial strategies to treat multi-drug resistant Staphylococcus aureus infections. Microbial Biotechnology 16:1456-1474.
3. Cui L, Murakami H, Kuwahara-Arai K, Hanaki H, Hiramatsu K. 2000. Contribution of a Thickened Cell Wall and Its Glutamine Nonamidated Component to the Vancomycin Resistance Expressed by Staphylococcus aureus Mu50. Antimicrobial Agents and Chemotherapy 44:2276-2285.
4. Potter AD, Butrico CE, Ford CA, Curry JM, Trenary IA, Tummarakota SS, Hendrix AS, Young JD, Cassat JE. 2020. Host nutrient milieu drives an essential role for aspartate biosynthesis during invasive Staphylococcus aureus infection. Proceedings of the National Academy of Sciences 117:12394-12401.
5. Mwangi MM, Kim C, Chung M, Tsai J, Vijayadamodar G, Benitez M, Jarvie TP, Du L, Tomasz A. 2013. Whole-genome sequencing reveals a link between β-lactam resistance and synthetases of the alarmone (p)ppGpp in Staphylococcus aureus. Microb Drug Resist 19:153-9.
6. Patil M, Serhii K, Garzino F, Gobert Q, Giorgio S, Raimundo J-M, Bolla J-M, Camplo M. 2023. Synthesis and antimicrobial testing of 5-fluorouracil derivatives. Archiv der Pharmazie 356:2300103.
7. Hignett RC. 1964. The incorporation of 5-fluorouracil by Staphylococcus aureus (strain Duncan). II. Biochimica et Biophysica Acta (BBA) - Specialized Section on Nucleic Acids and Related Subjects 91:584-591.
8. **Singh V, Brecik M, Mukherjee R, Evans JC, Svetlíková Z, Blaško J, Surade S, Blackburn J, Warner DF, Mikušová K, Mizrahi V.** 2015. The complex mechanism of antimycobacterial action of 5-fluorouracil. Chem Biol 22:63-75.
9. Longley DB, Harkin DP, Johnston PG. 2003. 5-Fluorouracil: mechanisms of action and clinical strategies. Nature Reviews Cancer 3:330-338.
10. Peters GJ. 2020. Chapter 1 - Drug resistance in colorectal cancer: General aspects, p 1-33. In Cho CH, Hu T (ed), Drug Resistance in Colorectal Cancer: Molecular Mechanisms and Therapeutic Strategies, vol 8. Academic Press.
11. Kilstrup M, Hammer K, Ruhdal Jensen P, Martinussen J. 2005. Nucleotide metabolism and its control in lactic acid bacteria. FEMS Microbiol Rev 29:555-90.
12. Kriegeskorte A, Block D, Drescher M, Windmuller N, Mellmann A, Baum C, Neumann C, Lore NI, Bragonzi A, Liebau E, Hertel P, Seggewiss J, Becker K, Proctor RA, Peters G, Kahl BC. 2014. Inactivation of thyA in Staphylococcus aureus attenuates virulence and has a strong impact on metabolism and virulence gene expression. mBio 5:e01447-14.
13. Neuville M, El-Helali N, Magalhaes E, Radjou A, Smonig R, Soubirou J-F, Voiriot G, Le Monnier A, Ruckly S, Bouadma L, Sonneville R, Timsit J-F, Mourvillier B. 2017. Systematic overdosing of oxa- and cloxacillin in severe infections treated in ICU: risk factors and side effects. Annals of Intensive Care 7:34.
14. Choo EJ, Chambers HF. 2016. Treatment of Methicillin-Resistant Staphylococcus aureus Bacteremia. Infect Chemother 48:267-273.
15. Brown GR. 2014. Cotrimoxazole - optimal dosing in the critically ill. Ann Intensive Care 4:13.
16. Casale F, Canaparo R, Serpe L, Muntoni E, Pepa CD, Costa M, Mairone L, Zara GP, Fornari G, Eandi M. 2004. Plasma concentrations of 5-fluorouracil and its metabolites in colon cancer patients. Pharmacol Res 50:173-9.
17. Van Der Heyden SA, Highley MS, De Bruijn EA, Tjaden UR, Reeuwijk HJ, Van Slooten H, Van Oosterom AT, Maes RA. 1999. Pharmacokinetics and bioavailability of oral 5'-deoxy-5-fluorouridine in cancer patients. Br J Clin Pharmacol 47:351-6.
18. Barcia-Macay M, Lemaire S, Mingeot-Leclercq M-P, Tulkens PM, Van Bambeke F. 2006. Evaluation of the extracellular and intracellular activities (human THP-1 macrophages) of telavancin versus vancomycin against methicillin-susceptible, methicillin-resistant, vancomycin-intermediate and vancomycin-resistant Staphylococcus aureus. Journal of Antimicrobial Chemotherapy 58:1177-1184.
19. Schmidt AP, Böhmer AE, Schallenberger C, Antunes C, Tavares RG, Wofchuk ST, Elisabetsky E, Souza DO. 2010. Mechanisms involved in the antinociception induced by systemic administration of guanosine in mice. Br J Pharmacol 159:1247-63.
20. Hawas S, Verderosa AD, Totsika M. 2022. Combination Therapies for Biofilm Inhibition and Eradication: A Comparative Review of Laboratory and Preclinical Studies. Front Cell Infect Microbiol 12:850030.
21. Brunke MS, Konrat K, Schaudinn C, Piening B, Pfeifer Y, Becker L, Schwebke I, Arvand M. 2022. Tolerance of biofilm of a carbapenem-resistant Klebsiella pneumoniae involved in a duodenoscopy-associated outbreak to the disinfectant used in reprocessing. Antimicrobial Resistance & Infection Control 11:81.
22. Osland AM, Oastler C, Konrat K, Nesse LL, Brook E, Richter AM, Gosling RJ, Arvand M, Vestby LK. 2023. Evaluation of Disinfectant Efficacy against Biofilm-Residing Wild-Type Salmonella from the Porcine Industry. Antibiotics (Basel) 12.
23. Liebeke M, Dörries K, Meyer H, Lalk M. 2012. Metabolome analysis of gram-positive bacteria such as Staphylococcus aureus by GC-MS and LC-MS. Methods Mol Biol 815:377-98.
24. Zeden MS, Gallagher LA, Bueno E, Nolan AC, Ahn J, Shinde D, Razvi F, Sladek M, Burke Ó, O'Neill E, Fey PD, Cava F, Thomas VC, O'Gara JP. 2023. Metabolic reprogramming and altered cell envelope characteristics in a pentose phosphate pathway mutant increases MRSA resistance to β-lactam antibiotics. PLOS Pathogens 19:e1011536.
25. Bulock LL, Ahn J, Shinde D, Pandey S, Sarmiento C, Thomas VC, Guda C, Bayles KW, Sadykov MR. 2022. Interplay of CodY and CcpA in Regulating Central Metabolism and Biofilm Formation in Staphylococcus aureus. Journal of Bacteriology 204:e00617-21.
26. Doellinger J, Schneider A, Hoeller M, Lasch P. 2020. Sample Preparation by Easy Extraction and Digestion (SPEED) - A Universal, Rapid, and Detergent-free Protocol for Proteomics Based on Acid Extraction. Mol Cell Proteomics 19:209-222.
27. Alqarzaee AA, Chaudhari SS, Islam MM, Kumar V, Zimmerman MC, Saha R, Bayles KW, Frees D, Thomas VC. 2021. Staphylococcal ClpXP protease targets the cellular antioxidant system to eliminate fitness-compromised cells in stationary phase. Proceedings of the National Academy of Sciences 118:e2109671118.
28. Wayne P. CLSI 2018. CLSI 2018. Performance standards for antimicrobial disk susceptibility tests. Approved standard, 12th ed. CLSI standard M02-A13. Clinical and Laboratory Standards Institute,.
29. Durham PG, Sidders AE, Beam JE, Kedziora KM, Dayton PA, Conlon BP, Papadopoulou V, Rowe SE. 2021. Harnessing ultrasound-stimulated phase change contrast agents to improve antibiotic efficacy against methicillin-resistant Staphylococcus aureus biofilms. Biofilm 3:100049.

## Claims

1. An antibiotic composition comprising or consisting of:
a purine nucleoside selected from guanosine and xanthosine, or a combination thereof; and
an inhibitor of thymidine and/or pyrimidine biosynthesis.

2. The antibiotic composition according to claim 1, further comprising a β-lactam antibiotic.

3. The antibiotic composition according to claim 2, wherein the β-lactam antibiotic is selected from methicillin, oxacillin, nafcillin, cefoxitin, cefotaxime, cefaclor, penicillin G, and cloxacillin, or variants thereof.

4. The antibiotic composition according to claim 2 or 3, wherein the β-lactam antibiotic is provided in an amount of from 7% to 33 % w/v of the composition.

5. The antibiotic composition according to any of claims 2-4, wherein the β-lactam antibiotic, is provided in the composition at a concentration of from 25 mg/kg to 300 mg/kg.

6. The antibiotic composition according to any preceding claim, wherein the purine nucleoside is provided in an amount of from about 30% to about 90 % w/v of the composition.

7. The antibiotic composition according to any preceding claim, wherein the purine nucleoside may be provided in the composition at a concentration up to about 960mg/kg or 1g/L in solution.

8. The antibiotic composition according to any preceding claim, wherein the inhibitor of thymidine and/or pyrimidine biosynthesis comprises an agent that inhibits one or more of dihydroorotate dehydrogenase (DHODH), aspartate transcarbamoylase (ATCase), dihydrofolate reductase (DHFR), carbamoyl phosphate synthetase II (CPS II), thymidylate synthase (TS), orotindine 5'-monophosphate decarboxylase (ODCase), and folate metabolism.

9. The antibiotic composition according to any preceding claim, wherein the inhibitor of thymidine and/or pyrimidine biosynthesis is selected from 5-fluorouracil (5-FU), 5-fluorouridine (5-FUrd), sulfamethoxazole (SMX), trimethoprim (TMP), and combinations thereof, or variants thereof.

10. The antibiotic composition according to any preceding claim, wherein the inhibitor of thymidine and/or pyrimidine biosynthesis comprises a combination of sulfamethoxazole and trimethoprim (SMX-TMP).

11. The antibiotic composition according to any preceding claim, wherein the inhibitor of thymidine and/or pyrimidine biosynthesis is provided in the composition at a concentration of from about 7% to about 33% w/v.

12. The antibiotic composition according to any one of claims 2-11, wherein the β-lactam antibiotic, the purine nucleoside, and the inhibitor of thymidine or pyrimidine biosynthesis, are provided in a ratio from about 1:1:1 to about 1:14:1 (w/w).

13. A kit, wherein the kit comprises or consists of:
a) a purine nucleoside selected from guanosine and xanthosine, or a combination thereof; and
b) one or more inhibitors of thymidine and/or pyrimidine biosynthesis.

14. The kit according to claim 13, wherein the kit further comprises c) a β-lactam antibiotic.

15. An antibiotic composition according to any of claims 1-12, or a kit according to claims 13 or 14, for use as a medicament.

16. Use of the composition according to any of claims 1-12, or a kit according to claims 13 or 14 in the manufacture of a medicament.

17. The antibiotic composition or the kit according to claim 15, or the use according to claim 16, wherein the medicament is for use to treat or prevent a bacterial infection or colonisation of a subject.

18. A method of treatment or prevention of a bacterial infection or colonisation of a subject, the method comprising administration of the antibiotic composition according to the invention to the subject, or the administration of:
a) a purine nucleoside selected from guanosine and xanthosine, or a combination thereof; and
b) an inhibitor of thymidine and/or pyrimidine biosynthesis, to the subject.

19. The method according to claim 18, further comprising the administration of a β-lactam antibiotic.

20. A method of reducing resistance to a β-lactam antibiotic in a bacterium, the method comprising the administration of the composition according to any of claims 1-12, or the kit according to claims 13 or 14, to the bacterium.

21. A method of treatment or prevention of a bacterial biofilm, the method comprising the administration of the composition according to any of claims 1-12, or the kit according to claims 13 or 14, to the bacterial biofilm.

22. Use of the composition according to any of claims 1-12, or the kit according to claims 13 or 14, to kill or inhibit a targeted bacterium, and/or to reduce the number of viable bacteria in an infection, or biofilm, that includes a targeted bacterium.

23. A method of killing or inhibiting the growth of a targeted bacteria on a surface, object or in a liquid, the method comprising applying the composition according to any of claims 1-12, or the kit according to claims 13 or 14, to the surface, object or liquid.
